# EUROPEAN PATENT SPECIFICATION

(11) **EP 0 810 226 B1**
(45) Date of publication and mention of the grant of the patent: **02.05.2003**
(21) Application number: 96901986.8
(22) Date of filing: 13.02.1996
(51) Int. Cl.: C07D 409/06, A01N 43/56, C07D 333/54, C07D 335/06

(54) **PYRAZOLE DERIVATIVES**
PYRAZOL-DERIVATE
DERIVES DE PYRAZOLE

(30) Priority: 13.02.1995 JP 2410295
(43) Date of publication of application: 03.12.1997
(73) Proprietor: IDEMITSU KOSAN COMPANY LIMITED, Tokyo 100-0005 (JP)
(72) Inventor: NASUNO, Ichiro, Sodegaura-shi Chiba-ken 299-02 (JP); SAKAMOTO, Masashi, Sodegaura-shi Chiba-ken 299-02 (JP); NAKAMURA, Kazufumi, Sodegaura-shi Chiba-ken 299-02 (JP); KOIKE, Kazuyoshi, Sodegaura-shi Chiba-ken 299-02 (JP)
(74) Representative: Gille Hrabal Struck Neidlein Prop Roos
(86) International application number: JP9600298
(87) International publication number: WO96025412

(56) References cited:
- EP-A- 0 352 543
- EP-A- 0 629 623
- WO-A-95/04054
- WO-A-96/00008
- WO-A-97/08164
- WO-A-97/23135
- DE-A- 19 532 312

## Description

### Technical Field

The present invention relates to novel pyrazole derivatives, herbicides containing the pyrazole derivatives as active ingredients, and intermediates suitable for the production of the pyrazole derivatives.

### Technical Background and Problems which the Invention Seeks to Solve

Herbicides are very important chemicals for saving weed-controlling labors and improving the yield of agricultural and horticultural crops and have been therefore aggressively studied and developed for many years, and a diversity of herbicides has now been put to practical use. Today, however, it is still desired to develop novel chemicals having excellent herbicidal efficacy, particularly chemicals which can selectively control target weeds alone at a low dosage without causing phytotoxicity on crops.

During the time of planting corn, etc., a triazine-based herbicide such as atrazine and acid anilide-based herbicides such as alachlor and methlachlor have been conventionally used. However, atrazine shows low efficacy to gramineous weeds, and on the other hand, alachlor and methalachlor show low efficacy to broad-leaved weeds. It is therefore difficult at present to control gramineous weeds and broad-leaved weeds together simultaneously with a single herbicide. Further, the above herbicides are undesirable in view of an environmental problem due to their high dosage requirement.

It is known that in paddy land, a variety of weeds, annual gramineous weeds such as barnyardgrass, annual cyperaceous weeds such as umbrella plant, annual broad-leaved weeds such as monochoria and toothcup and perennial weeds such as sagitaria pygmaea, pondweed, oriental water plantain, bulrush, needle spikerush, serotinus, water chestnut, arrowhead and dropwort grow together with paddy rice, and in rice cultivation, it is very important to effectively control these weeds at a low dosage in view of environmental pollution without causing phytotoxicity on paddy rice. Generally, it is known that chemicals having high herbicidal efficacy on barnyardgrass are liable to cause phytotoxicity on paddy rice, and it is a particularly important subject to develop a chemical which has high herbicidal efficacy on barnyardgrass as a gramineous weed and has excellent inter-genus selectivity between paddy rice and barnyardgrass.

Meanwhile, it is known that specific 4-benzoylpyrazoles have herbicidal efficacy (JP-A-63-122672, JP-A-63-122673, JP-A-63-170365, JP-A-1-52759, JP-A-2-173 and JP-A-2-288866), and pyrazolate of the following chemical formula is among herbicides commercially available at present.

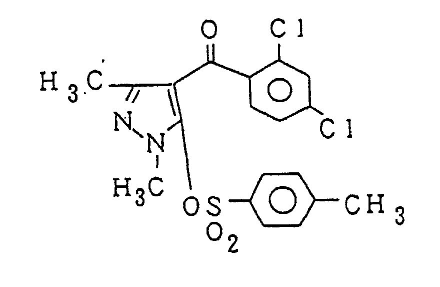

Further, a compound (A), the typical example of 4-benzoylpyrazole derivatives disclosed in the above publications, has the following chemical formula (Compound No. 35 in JP-A-2-173).

### Compound (A): Compound No. 35 disclosed in JP-A-2-173

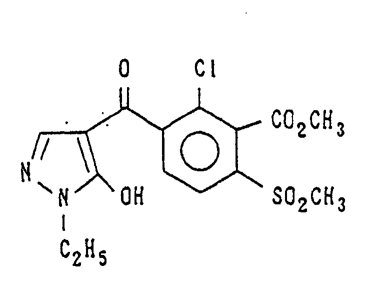

The above 4-benzoylpyrazole derivatives have herbicidal activity, while their herbicidal activity is practically insufficient. In particular, their herbicidal activity to gramineous weeds such as barnyardgrass and green foxtail is very poor. When they are used as a herbicide for controlling weeds in paddy land, they may cause phytotoxicity on paddy rice since they have poor selectivity between paddy rice and gramineous weeds.

The present inventors have therefore proposed pyrazole derivatives having a thiochroman ring and have filed patent applications directed thereto (JP-A-4-185526 and International Laid-open Patent Publication WO93/18031).

Pyrazole derivatives with a benzothiopyran structure and displaying a herbicidal activity are described in EP -A- 629 623, EP - A- 0 712 853, WO96/00008 and WO97/23135.-Pyrazol-4-yl benzoyl derivatives with herbicidal activity are described in DE -A- 195 32 312 and WO97/08164.

Typical examples (B) and (C) of the compounds disclosed in the specifications of the above prior applications are as follows.

### Compound (B): Compound No. 66 disclosed in International Laid-open Patent Publication WO93/18031

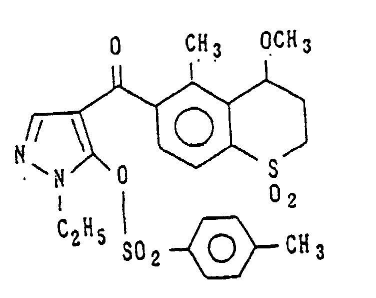

### Compound (C): Compound No. b-3 disclosed in JP-A-4-185526

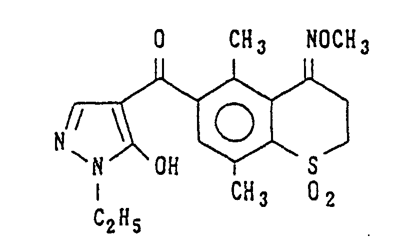

The above compounds have high herbicidal activity, while they still have room for improvement in view of safety to paddy rice.

The present invention has been made in view of the above circumstances, and the object thereof is to provide a pyrazole derivative which can control a broad range of upland weeds and paddy land weeds, particularly barnyardgrass in paddy land, at a low dosage without causing phytotoxicity on crops such as corn, paddy rice, etc., a herbicide containing the same, and an intermediate for obtaining the pyrazole derivative.

### Disclosure of the Invention

The first gist of the present invention is directed to a pyrazole derivative of the formula (I-A), 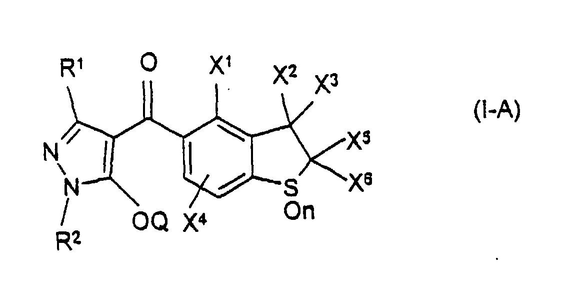 wherein:
R¹ is a hydrogen atom or a C₁-C₄ alkyl group;
R² is a C₁-C₄ alkyl group;
each of X² and X³ is independently one member selected from the group consisting of a hydrogen atom and a C₁-C₄ alkyl group;
each of X¹ and X⁴ is independently one member selected from the group consisting of a hydrogen atom, a halogen atom and a C₁-C₄ alkyl group;
each of X⁵ and X⁶ is independently a hydrogen atom or a C₁-C₄ alkyl group or X² and X⁵ may form an unsaturated bond by bonding to each other,
Q is a hydrogen atom or a group of -A-B in which A is one member selected from the group consisting of -SO₂-, -CO- and -CH₂CO-, and
B is one member selected from the group
consisting of a C₁-C₈ alkyl group, a C₃-C₈ cycloalkyl group and a group of the formula (V), 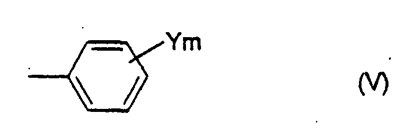 in which Y is one member selected from the group consisting of a halogen atom, a nitro group, a C₁-C₄ alkyl group, a C₁-C₄ alkoxy group and a C₁-C₄ haloalkyl group, and m is an integer of 0, 1 or 2 ; and n is 0, 1 or 2 , or a salt thereof.

The second gist of the present invention is directed to a herbicide containing a pyrazole derivative of the above formula (I-A) and/or a salt thereof as an active ingredient.

Further, the third gist of the present invention is directed to an aromatic carboxylic acid derivative of the frormula (II-A) useful for the production of the pyrazole derivative of the above formula (I-A), 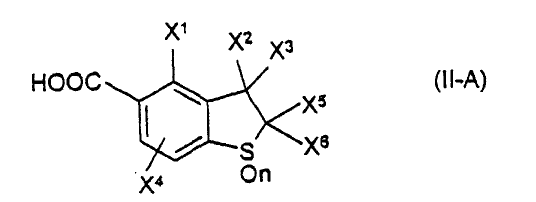 wherein:
each of X¹ and X⁴ is independently a halogen atom or a C₁-C₄ alkyl group;
each of X² and X³ is independently one member selected from the group consisting of a hydrogen atom and a C₁-C₄ alkyl group;
each of X⁵ and X⁶ is independently a hydrogen atom or a C₁-C₄ alkyl group, or X² and X⁵ may form an unsaturated bond by bonding to each other; and n is 0, 1 or 2 ,
or a salt thereof.

### Preferred Embodiments for Working the Invention

The novel pyrazole derivative of the present invention is a compound of the formula (I-A),

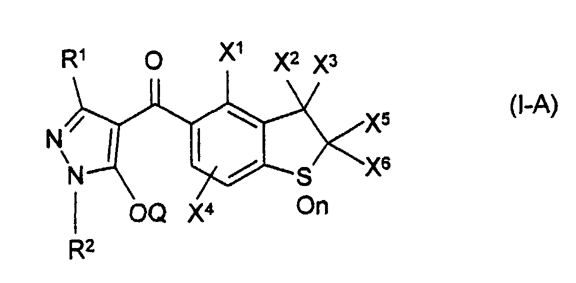

In the formula (I-A), R¹ is a hydrogen atom or a C₁∼C₄ alkyl group, preferably a hydrogen atom. R² is a C₁∼C₄ alkyl group. The C₁∼C₄ alkyl group as R¹ and R² each includes methyl, ethyl, propyls such as n-propyl and i-propyl, and butyls such as n-butyl and i-butyl. The C₁∼C₄ alkyl group as R² is preferably ethyl.

X¹ is one member selected from the group consisting of a hydrogen atom, a halogen atom and a C₁∼C₄ alkyl group. The halogen atom as X¹ includes fluorine, chlorine, bromine and iodine. The C₁∼C₄ alkyl group as X¹ includes those described concerning R¹ and R². X¹ is preferably a C₁∼C₄ alkyl group, more preferably methyl.

Each of X⁵ and X⁶ is independently a hydrogen atom or a C₁∼C₄ alkyl group, or X² and X⁵ may constitute an unsaturated bond by bonding to each other. The C₁∼C₄ alkyl group includes those described concerning R¹ and R². Preferably, each of these substituents is independently a hydrogen atom, methyl or ethyl, or X² and X⁵ constitute a double bond.

X⁴ is one member selected from the group consisting of a hydrogen atom, a halogen atom and a C₁∼C₄ alkyl group. The halogen atom includes those described concerning X¹, and the C₁∼C₄ alkyl group includes those described concerning R¹ and R². X⁴ is preferably a hydrogen atom or a C₁∼C₄ alkyl group, more preferably methyl. The position on which X⁴ is substituted is preferably the 7-position on a benzo[b]thiophene ring.

Q is a hydrogen atom or a group of the formula -A-B, in which A is one member selected from the group consisting of -SO₂-, -CO- and -CH₂CO-, and B is one member selected from the group consisting of a C₁∼C₈ alkyl group, a C₃-C₈ cycloalkyl group and a group of the formula (V).

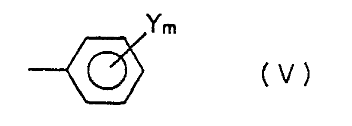

The C₁∼C₈ alkyl group as one embodiment of B includes methyl, ethyl, propyl, butyl, pentyl, hexyl, heptyl and octyl, and an alkyl group having at least 3 carbon atoms may be linear or branched. Preferred is ethyl, n-propyl or i-propyl. The C₃-C₈ cycloalkyl group as another embodiment of B includes cyclopropyl, cyclobutyl, cyclopentyl, cyclohexyl, cycloheptyl and cyclooctyl, and cyclohexyl is preferred.

Further, in the group of the formula (V) as another embodiment of B, Y is a halogen atom, a nitro group, a C₁∼C₄ alkyl group, a C₁∼C₄ alkoxy group or a C₁∼C₄ haloalkyl group. The halogen atom includes those described concerning X¹ and X⁴. The C₁∼C₄ alkyl group includes those described concerning R¹ and R², and methyl is preferred. The C₁∼C₄ alkoxy group includes methoxy, ethoxy, linear or branched propoxy and linear or branched butoxy. The C₁∼C₄ haloalkyl group includes those obtained by replacing hydrogen atoms of the C₁∼C₄ alkyl group described concerning R¹ and R² with halogen atoms described concerning X¹ and X⁴. Specifically, the C₁∼C₄ haloalkyl group includes -CH₂F, -CHF₂, -CF₃, -CF₂CF₃, -CCl₃ and -CH₂CF₃.

m which means the number of Y is an integer of 0, 1 or 2.

One preferred embodiment of a combination of A with B of the group of -A-B is, for example, that when A is -SO₂-, B is a C₁∼C₈ alkyl group or a phenyl group sustituted with 1 or 2 halogen atom(s), nitro group(s), C₁∼C₄ alkyl group(S) or C₁∼C₄ alkoxy group(s) (a group of the formula (V) in which Y is a halogen atom, a nitro group, a C₁∼C₄ alkyl group or a C₁∼C₄ alkoxy group and m is 1 or 2). Another preferred embodiment of a combination of A with B of the group of -A-B is that when A is -CO- or -CH₂CO-, B is a C₁∼C₈ alkyl group or a halogen-substituted or halogen-nonsubstituted phenyl group (a group of the formula (V) in which Y is a halogen atom and m is 0, 1 or 2).

n is the number of oxygen, and is an integer of 0, 1 or 2. That is, when n is 0, a sulfide is represented. When n is 1, a sulfoxide is represented. When n is 2, a sulfone is represented.

The pyrazole derivative of the general formula (I-A) in which Q is hydrogen, i.e., a compound of the formula (I-Aa) 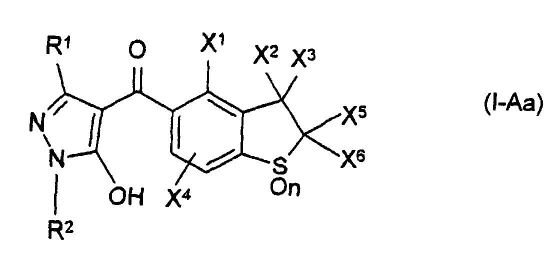 can have the following three structures due to tautomerism, and the pyrazole derivative of the present invention includes all of the above structures.

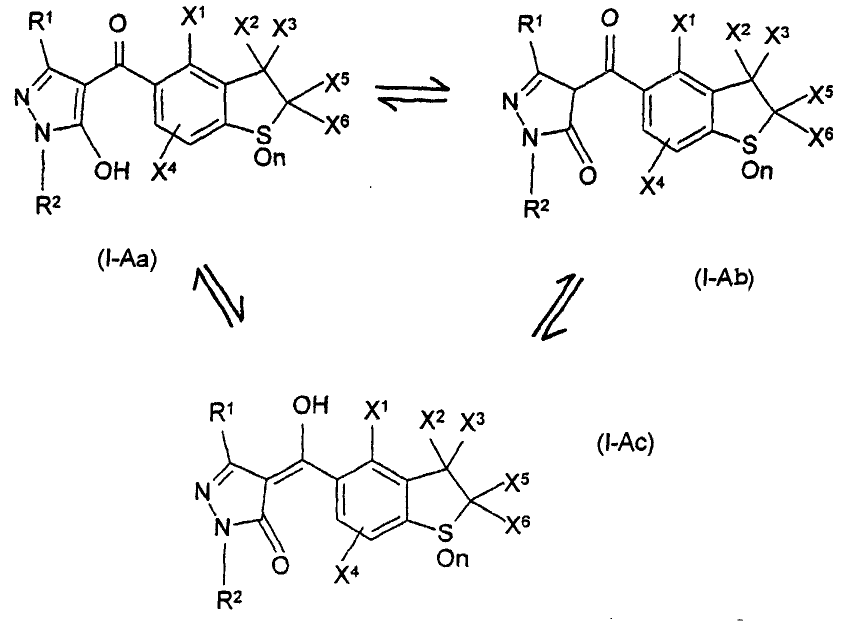

Further, the pyrazole derivative of the formula (I-Aa) is an acidic substance, and can be easily converted to a salt by treating it with a base. The pyrazole derivative of the present invention also includes the salt. The base can be selected from known bases without any problem, while examples of the base include organic bases such as amines and anilines and inorganic bases such as sodium compounds and potassium compounds. The amines include monoalkylamine, dialkylamine and trialkylamine. The alkyl group of the alkylamines is generally a C₁∼C₄ alkyl group. The anilines include aniline, monoalkylaniline and dialkylaniline. The alkyl group of the alkylalines is generally a C₁∼C₄ alkyl group. The sodium compounds include sodium hydroxide and sodium carbonate, and the potassium compounds include potassium hydroxide and potassium carbonate.

The herbicide of the present invention contains the novel pyrazole derivative of the formula (I-A) and/or a salt thereof as an active ingredient. The herbicide can be prepared by mixing these compounds with a liquid carrier such as a solvent or a solid carrier such as a mineral fine powder, forming the mixture into the preparation form of a wettable powder, an emulsifiable concentrate, a dust or granules. The herbicide can be imparted with emulsifiability, dispersibility and spreadability by adding a surfactant when the herbicide is prepared.

When the herbicide of the present invention is used in the form of a wettable powder, generally, a composition prepared by mixing 10 to 55 % by weight of the pyrazole derivative and/or its salt of the present invention, 40 to 88 % by weight of a solid carrier and 2 to 5 % by weight of a surfactant can be used as a wettable powder. Further, when it is used in the form of an emulsifiable concentrate, generally, it can be prepared by mixing 20 to 50 % by weight of the pyrazole derivative and/or its salt of the present invention, 35 to 75 % by weight of a solvent and 5 to 15 % by weight of a surfactant.

On the other hand, when it is used in the form of a dust, generally, it can be prepared by mixing 1 to 15 % by weight of the pyrazole derivative and/or its salt of the present invention, 80 to 97 % by weight of a solid carrier and 2 to 5 % by weight of a surfactant. Further, when it is used in the form of granules, the granules can be prepared by mixing 1 to 15 % by weight-of the pyrazole derivative and/or its salt of the present invention, 80 to 97 % by weight of a solid carrier and 2 to 5 % by weight of a surfactant.

The above solid carrier can be selected from mineral fine powders, and the mineral fine powders include oxides such as diatomaceous earth and slaked lime, phosphates such as apatite, sulfates such as gypsum, and silicates such as talc, pyroferrite, clay, kaolin, bentonite, acid clay, white carbon, powdered quartz and powdered silica.

The solvent is selected from organic solvents, and specific examples of the organic solvents include aromatic hydrocarbons such as benzene, toluene and xylene, chlorinated hydrocarbons such as o-chlorotoluene, trichloroethane and trichloroethylene, alcohols such as cyclohexanol, amyl alcohol and ethylene glycol, ketones such as isophorone, cyclohexanone and cyclohexenyl-cyclohexanone, ethers such as butyl cellosolve, diethyl ether and methyl ethyl ether, esters such as isopropyl acetate, benzyl acetate and methyl phthalate, amides such as dimethylformamide, and mixtures of these.

Further, the surfactant can be selected from anionic, nonionic, cationic and amphoteric ones (amino acid and betaine).

In combination with the pyrazole derivative of the above general formula (I) and/or the salt thereof, the herbicide of the present invention may contain, as active ingredients, other herbicidally active component as required. The "other" herbicidally active component can be selected from known herbicides such as phenoxy-, diphenyl ether-, triazine-, urea-, carbamate-, thiocarbamate-, acid anilide-, pyrazole-, phosphoric acid-, sulfonylurea- and oxadiazone-based herbicides. The other herbicidally active component can be properly selected from the above herbicides.

Further, the herbicide of the present invention may contain an insecticide, a fungicide, a plant growth regulator and a fertilizer as required.

The pyrazole derivative of the formula (I-A), provided by the present invention, is produced by the following methods (1) and (2).

First, the method (1) of producing the pyrazole derivative of the present invention will be explained in detail.

### Method (1) of producing pyrazole derivative

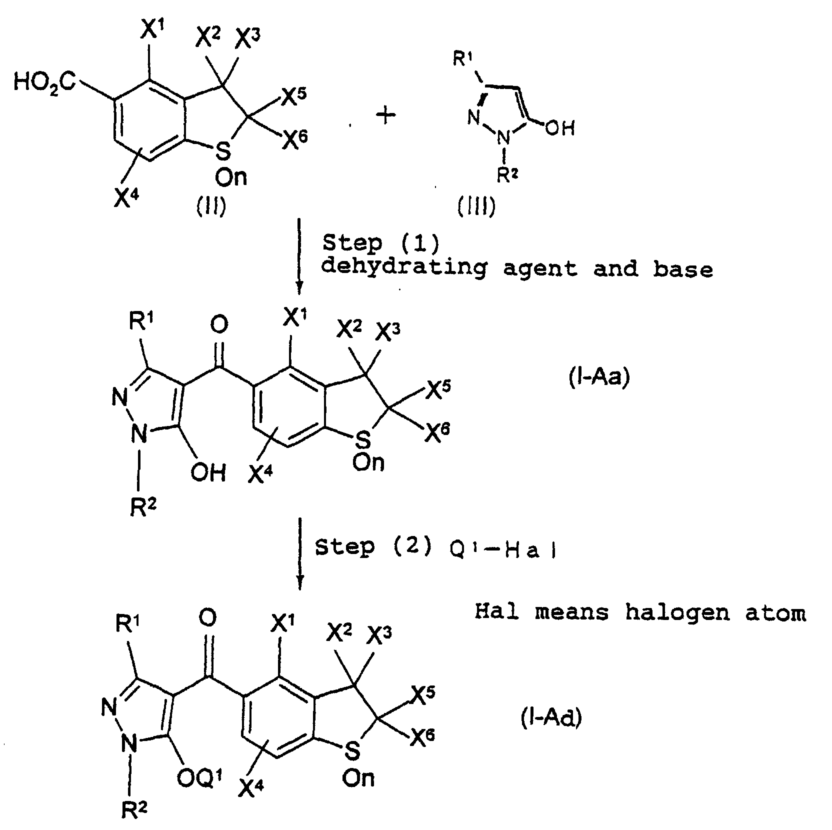

In the above reaction scheme, Q¹ is a group of -A-B in which A is one member selected from the group consisting of -SO₂-, -CO- and -CH₂CO-, B is one member selected from the group consisting of a C₁∼C₈ alkyl group, a C₃-C₈ cycloalkyl group and a group of the formula, 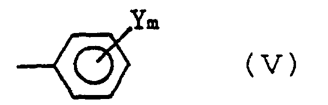 wherein Y is one member selected from the group consisting of a halogen atom, a nitro group, a C₁∼C₄ alkyl group, a C₁∼C₄ alkoxy group and a C₁∼C₄ haloalkyl group, and m represents the number of Y and is an integer of 0, 1 or 2. Further, R¹, R², X¹, X², X³, X⁴, X⁵, X⁶, X⁷, X⁸, p and n are as previously defined in the general formula (I), and Hal is a halogen atom.

The above production method will be explained step by step hereinafter.

### (Step 1)

A compound of the formula (II) and a compound of the formula (III) are reacted with each other in an inert solvent in the presence of a dehydrating agent such as DCC (N,N'-dicyclohexylcarbodimimide), CDI (1,1-carbonyldiimidazole) or EDC (1-(3-dimethylaminopropyl)-3-ethylcarbodiimide) and a base, to produce a pyrazole derivative of the formula (Ia).

The amount of the compound of the formula (III) is preferably 1.0 ∼ 3.0 mol per mole of the compound of the formula (II). Further, the amount of the dehydrating agent is preferably 1.0 ∼ 1.5 mol per mole of the compound of the formula (II). The base is not specially limited in kind, while potassium carbonate or sodium carbonate is preferred. The amount of the base is preferably 0.5 to 2.0 mol per mole of the compound of the formula (II). The solvent for the reaction is not specially limited if it is inert to the reaction. The solvent is preferably selected from acetonitrile, 1,4-dioxane, t-amyl alcohol, t-butyl alcohol and i-propyl alcohol. The reaction temperature can be selected from the range of from 0°C to the boiling point of the solvent, while it is preferably about 80°C. The reaction time is 1 ∼ 48 hours, while it is generally about 8 hours.

An ester is formed as a reaction intermediate, and this ester intermediate can be isolated by means such as silica gel column chromatography, while the reaction is generally proceeded with without isolating the ester intermediate. When the ester intermediate is isolated, a base is added to the ester intermediate to further proceed with the reaction, whereby the intended pyrazole derivative (Ia) can be obtained. The amount of the base used in this case is 0.5 to 3.0 equivalents, preferably 0.5 to 1.5 equivalents, based on the ester intermediate. The reaction temperature is generally 80 ∼ 150°C, preferably 100 ∼ 120°C. The reaction time is generally 0.5 to 8 hours, preferably approximately 1 to 2 hours.

After the completion of the reaction, according to a conventional method, the solvent is distilled off, the residue is liquid-separated with an organic solvent and water, the aqueous layer is neutralized with an acid such as hydrochloric acid and extracted with ethyl acetate, the organic layer is dried over a dehydrating agent such as anhydrous sodium sulfate, and the solvent is distilled off, whereby the intended pyrazole derivative (I-Aa) can be obtained.

### (Step 2)

The compound (I-Aa) obtained in step 1 is reacted with Q¹-Hal (IV) (in which Q¹ and Hal are as defined already) in an inert solvent in the presence of a base, to produce a compound (Id).

In this step, the compound (I-Aa):compound (IV) molar ratio is preferably 1:1 ∼ 1:3. For collecting hydrogen halide byproducted by the reaction, preferably, a base such as sodium carbonate, potassium carbonate, triethylamine or pyridine is usedin at least an equimolar amount based on the starting material of the formula (I-Aa). The reaction temperature is preferably set in the range of from room temperature to the boiling point of the solvent. The solvent used in the reaction includes aromatic hydrocarbons such as benzene and toluene, ethers such as diethyl ether, ketones such as methyl ethyl ketone and halogenated hydrocarbons such as methylene chloride and chloroform. A two-phase solvent consisting of the above solvent and water may be also used. In this case, a more desirable result can be obtained by adding a phase-transfer catalyst such as crown ether or benzyltriethylammonium chloride to the reaction system.

After the completion of the reaction, according to a conventional method, the reaction mixture is liquid-separated, the end product is extracted from the aqueous layer by means of an organic solvent such as dichloromethane, the organic layer is dehydrated and then solvent is distilled off, whereby the intended pyrazole derivative (Id) can be isolated.

The aromatic carboxylic acid derivative of the formula (II-A) used for the reaction with the compound of the formula (III) in the above method, 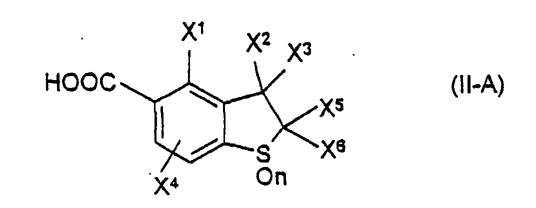 wherein:
each of X¹ and X⁴ is independently a halogen atom or a C₁-C₄ alkyl group;
each of X² and X³ is independently one member selected from the group consisting of a hydrogen atom and a C₁-C₄ alkyl group;
each of X⁵ and X⁶ is independently a hydrogen atom or a C₁-C₄ alkyl group, or X² and X⁵ may form an unsaturated bond by bonding to each other, and n is 0, 1 or 2,
is a novel compound described in no literature, and the aromatic carboxylic acid derivative of the formula (II-A) is useful as an intermediate for the production of the pyrazole derivative of the present invention.

Specific examples of X¹, X², X³, X⁴, X⁵ and X⁶ in the formula (II-A) include those described concerning the pyrazole derivative of the above formula (I).

The aromatic carboxylic acid derivative of the formula (II-A) is an acidic substance, and can be easily converted to a salt by treating it with a base. This salt is also included in the aromatic carboxylic acid derivative of the present invention. The base can be selected from known bases without any limitation. For example, the base includes organic bases such as amines and anilines and inorganic bases such as sodium compounds and potassium compounds. The amines include monoalkylamine, dialkylamine and trialkylamine. The alkyl group of the alkylamines is generally a C₁∼C₄ alkyl group. The anilines include aniline, monoalkylaniline and dialkylaniline. The alkyl group of the alkylalines is generally a C₁∼C₄ alkyl group. The sodium compounds include sodium hydroxide and sodium carbonate, and the potassium compounds include potassium hydroxide and potassium carbonate.

An aromatic carboxylic acid derivative of the general formula (II-A) (provided that X² and X⁵ do not bond to each other), i.e, an aromatic carboxylic acid derivative of the general formula (IIy), is produced by any one of methods in the following Production scheme 7 ∼ 10.

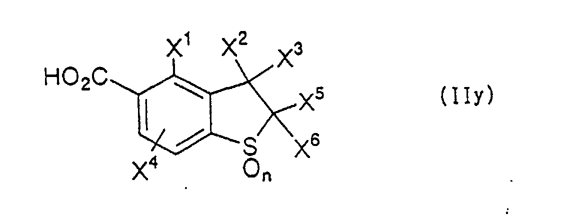

### Production scheme 7

Production scheme 7 shows a method of producing an aromatic carboxylic acid derivative of the general formula (IIy) in which X² is methyl.

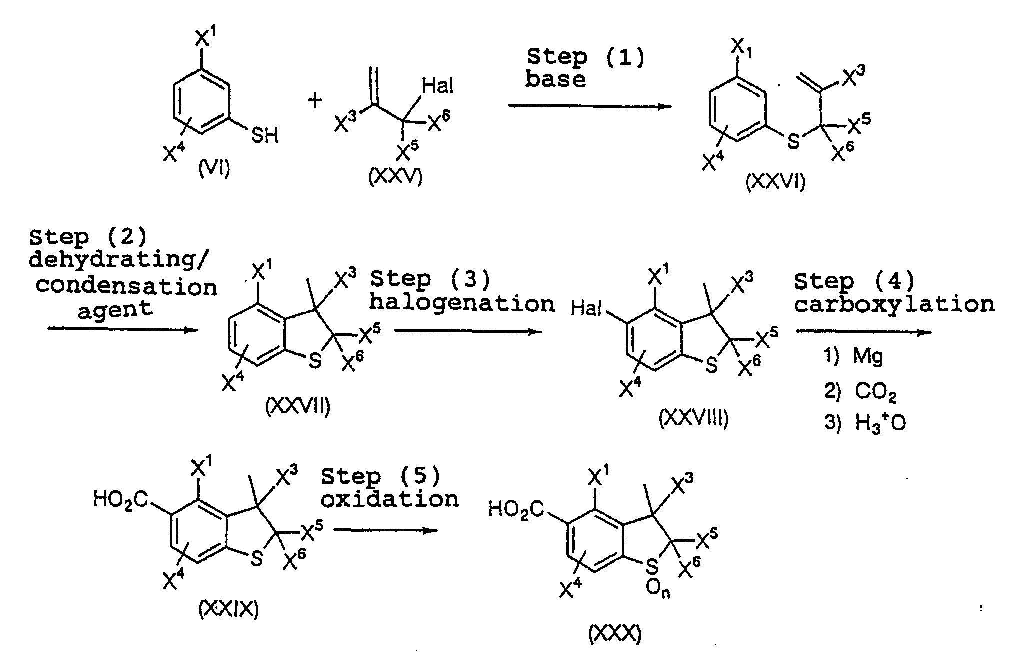

### (Step 1)

Step 1 is directed to a reaction in which a substituted thiphenol (VI) is reacted with a halogenated olefin (XXV) as an alkylating agent in the presence of a base, to obtain an alkyl-sbustituted thiophenol (XXVI).

The base used in the above reaction is selected from inorganic bases such as anhydrous potassium carbonate, sodium hydroxide and potassium hydroxide and organic bases such as triethylamine, while preferred is anhydrous potassium carbonate. The amount of the base is generally 0.5 ∼ 3.0 equivalents, preferably 1.0 ∼ 1.2 equivalents, based on the substituted thiophenol (VI).

The amount of the halogenated olefin (XXV) used as an alkylating agent in the above reaction is generally 1.0 ∼ 2.0 equivalents, preferably 1.0 ∼ 1.2 equivalents, based on the substituted thiophenol (VI).

Any solvent is used for the reaction without any special limitation if it is inert to the reaction, while acetone or dimethylformamide (DMF) is preferred. The reaction time is 10 minutes ∼ 8 hours, while it is generally completed in about 2 hours. The reaction temperature can be set in the range of 0°C ∼ the reflux temperature, while the range of from room temperature to 60°C is preferred.

After the completion of the reaction, the reaction mixture is cooled, insolubles are removed, and the solvent is distilled off. The residue is re-dispersed in an organic solvent such as hexane, washed and dried, and the solvent is distilled off, whereby the alkyl-substituted thiophenol (XXVI) can be isolated.

### (Step 2)

The step 2 is directed to a reaction for cyclizing the alkyl-substituted thiophenol (XVII) by an intra-molecular Friedel-Crafts reaction in the presence of a dehydration-condensation agent, to form a hydrobenzo[b]thiophene compound (XXVII).

The dehydration-condensation agent used in the above reaction is selected, for example, from sulfuric acid, phosphoric acid, phosphorus pentoxide and polyphosphoric acid, and polyphosphoric acid is preferred. The amount of the dehydration-condensation agent is generally 1 ∼ 10 mol equivalent based on the alkyl-substituted thiophenol (XXVI).

The reaction temperature is in the range of from room temperature to 200°C, while it is generally preferably 100 to 150°C. The reaction time is 30 minutes to 16 hours, while it is generally preferably 2 to 8 hours.

After the completion of the reaction, the reaction mixture is poured into ice water, a solvent such as hexane is added, and the mixture is liquid-separated. The resultant organic layer is washed and dehydrated, and then the solvent is distilled off. The resultant residue is purified by means such as column chromatography using a developer solvent such as hexane, whereby the hydrobenzo[b]thiophene compound (XXVII) can be isolated.

### (Step 3)

The step 3 is directed to a reaction for obtaining a halogenated benzo[b]thiophene compound (XXVIII) in which a halogen is substituted on the 5-position of the benzo[b]thiophene ring, by reacting the hydrobenzo[b]thiophene compound (XXVII) with a halogenating reagent such as bromine, sulfuryl chloride or chlorine in the presence of a solvent such as methylene chloride, chloroform or carbon tetrachloride.

The amount of the halogenating reagent used for the above reaction is generally 1.0 ∼ 3.0 equivalents, preferably 1.0 ∼ 1.5 equivalents, based on the hydrobenzo[b]thiophene compound (XXVIII). Preferably, the reaction temperature is generally 0 ∼ 80°C, and the reaction time is generally approximately 1 ∼ 80 hours.

After the completion of the reaction, excessive halogenating reagent is removed with a sodium hydrogensulfite aqueous solution and the residue is post-treated according to a conventional method, whereby the intended halogenated benzo[b]thiophene compound (XXVIII) can be isolated.

### (Step 4)

The step 4 is directed to a reaction for obtaining an aromatic carboxylic acid derivative of the formula (IIy) in which a carboxyl group is introduced onto the 5-position of a hydrobenzo[b]thiophene ring, a compound (XXIX) (n = 0, sulfide compound), by reacting the halogenated benzo[b]thiophene (XXVIII) with magnesium (Mg) to form a Grignard reagent and reacting the Grignard reagent with carbon dioxide (CO₂). It is preferred to use an ether such as diethyl ether or tetrahydrofuran as a solvent. The reaction temperature is 0 ∼ 70°C, particularly preferably 20 ∼ 60°C. The reaction time is generally approximately 1 ∼ 7 hours.

The amount of magnesium (Mg) for obtaining the Grignard reagent is preferably 1.1 ∼ 3.5 mol equivalent based on the halogenated hydrobenzo[b]thiophene compound (XXVIII). The Grignard reaction is preferably carried out in the co-presence of alkyl iodide such as methyl iodide or alkyl bromide such as ethyl bromide, since the reaction proceeds smoothly. The amount of the alkyl halide used in this case is preferably 0.1 ∼ 2.5 mol equivalent based on the halogenated hydrobenzo[b]thiophene compound (XXVIII).

The reaction between the Grignard reagent and carbon dioxide (CO₂) is carried out by introducing carbon dioxide gas from a gas container into the Grignard reagent in a solvent or by introducing carbon dioxide gas generated from dry ice (solid carbon dioxde). The reaction may be carried out by adding dry ice directly to the Grignard reagent.

After the reaction, an acid such as hydrochloric acid is added to the reaction mixture to terminate the reaction, the reaction mixture is liquid-separated by adding an organic solvent such as ethyl acetate, the resultant organic layer is liquid-separated by adding an alkali such as a potassium carbonate aqueous solution, and the resultant aqueous layer is neutralized with an acid such as hydrochloric acid and extracted with an organic solvent such as ethyl acetate. The resultant organic layer is washed and dried and then the solvent is distilled off, whereby the aromatic carboxylic acid derivative of the formula (IIy), a compound (XXIX) (n = 0, sulfide), can be isolated.

### (Step 5)

The step 5 is directed to a reaction for obtaining an aromatic carboxylic acid derivative of the formula (IIy), a compound (XXX) (n = 1, sulfoxide, n = 2, sulfone), by reacting an oxidizing agent (e.g., hydrogen peroxide, peracetic acid or sodium metaperiodate) with the compound of the formula (XXIX) in a solvent (e.g., acetic acid, water or methanol).

When the oxidizing agent in an amount of 1 equivalent based on the compound (XXIX) is reacted, a sulfoxide (a compound of the formula (IIy) in which n = 1) is obtained. When the oxidizing agent in an amount of at least 2 equivalents based on the compound (XXIX) is reacted, a sulfone (a compound of the formula (IIy) in which n = 2) is obtained.

In the above reaction, the reaction temperature is generally 25 ∼ 110°C, preferably 60 ∼ 100°C. The reaction time is generally 30 minutes ∼ 8 hours, preferably 1 ∼ 3 hours.

After the completion of the reaction, the reaction mixture is poured into a sodium hydrogensulfite aqueous solution and the mixture is liquid-separated by adding an organic solvent such as ethyl acetate. The resultant organic layer is washed and dried, and then the solvent is distilled off, whereby the intended compound of the formula (XXX) can be isolated.

### Production scheme 8

The production scheme 8 also shows a method of producing an aromatic carboxylic acid derivative of the general formula (IIy) in which X² is methyl.

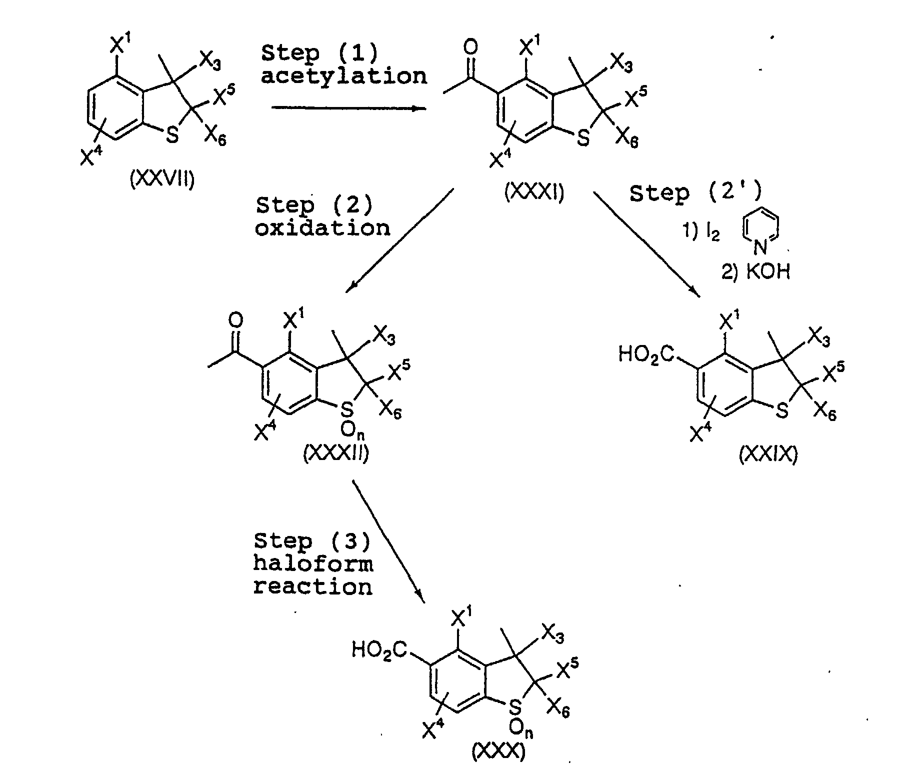

A hydrobenzo[b]thiophene compound of the formula (XXVII) as a starting material is obtained by steps 1 and 2 in Production scheme 7. Reactions in steps 1, 2, 2' and 3 are essentially the same as those in steps 3, 4, 4' and 5 in Production scheme 2, and their details are therefore omitted.

### Production scheme 9

Production scheme 9 shows a method of producing an aromatic carboxylic acid derivative of the general formula (IIy) in which both X² and X⁶ are hydrogen.

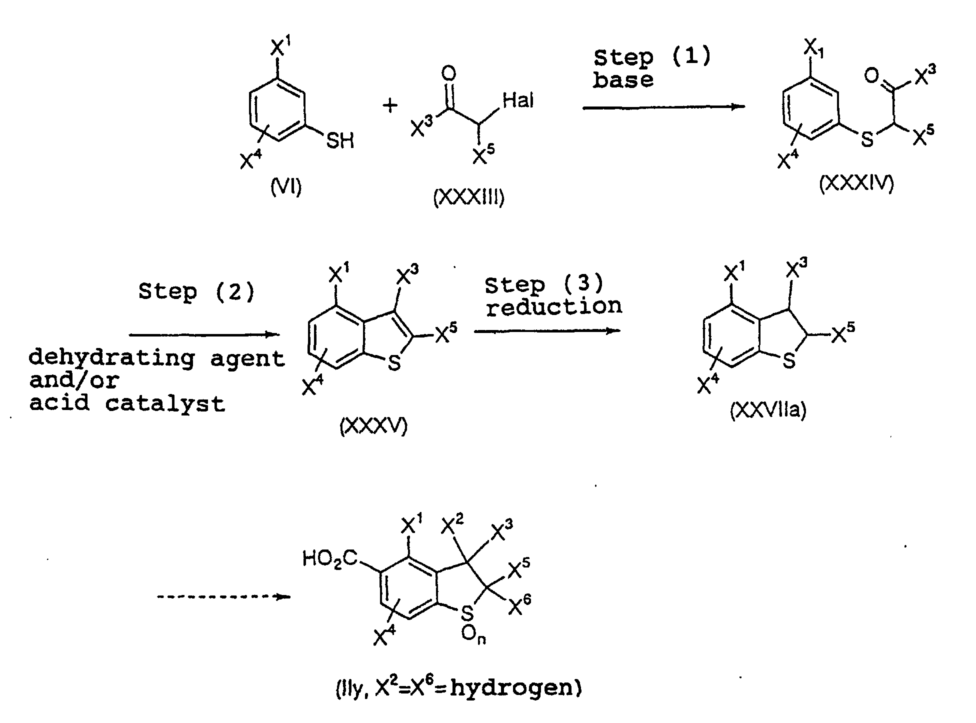

### (Step 1)

The step 1 is directed to a reaction for obtaining a compound of the formula (XXXIV) by condensing the substituted thiophenol (VI) and an α-halo-carbonyl compound (XXXIII) in the presence of a base.

The base used in the above reaction is selected from inorganic bases such as anhydrous potassium carbonate, sodium hydroxide and potassium hydroxide and organic bases such as triethylamine. Anhydrous potassium carbonate is preferred. The amount of the base is generally 0.5 ∼ 3.0 mol equivalent, preferably 1.0 ∼ 1.2 mol equivalent, based on the substituted thiophenol.

The amount of the α-halo-carbonyl compound (XXXIII) is generally 1.0 ∼ 2.0 mol equivalent, properly 1.0 ∼ 1.2 mol equivalent, based on the substantially thiophenol (VI).

Any solvent can be used without any special limitation if it is inert to the reaction, while it is proper to use acetone or dimethylformamide (DMF).

The reaction temperature can be set in the range of from 0°C to the reflux temperature of the solvent, while the range of from room temperature to 60°C is preferred. The reaction time is 10 minutes to 8 hours, while the reaction is generally completed in about 2 hours.

After the completion of the reaction, the reaction mixture is cooled, insolubles are removed and the solvent is distilled off. The resultant residue is re-dispersed in a solvent such as hexane, washed and dried, and the solvent is distilled of, whereby the compound (XXXIV) can be isolated.

### (Step 2)

The step 2 is directed to a reaction for forming a benzo[b]thiophene compound (XXXV) by subjecting the compound (XXXIV) obtained in step 1 to an intra-molecular dehydration-condensation reaction in the presence of a dehydrating agent and/or an acid catalyst.

The dehydrating agent used in the above reaction includes sulfuric acid, phosphoric acid, phosphorus pentoxide and polyphosphoric acid, and polyphosphoric acid is preferred. The acid catalyst includes p-toluenesulfonic acid, methanesulfonic acid and trifluoromethanesulfonic acid, and trifluoromethanesulfonic acid is preferred. The amount of the dehydrating agent and/or the acid catalyst is generally 1 ∼ 10 mol equivalent, preferably 1.0 ∼ 3.0 mol equivalent, based on the compound (XXXIV).

The reaction temperature can be set in the range of from 0°C to the reflux temperature of the solvent, while the range of from room temperature to 60°C is generally preferred. The reaction time is 10 minutes to 8 hours, while the reaction is generally completed in about 2 hours.

After the completion of the reaction, according to a conventional method, the reaction mixture is poured into ice water, a precipitated white crystal is re-dispersed in an organic solvent such as n-hexane, the dispersion is washed and the then solvent is distilled off, whereby the benzo[b]thiophene compound (XXXV) can be isolated.

### (Step 3)

The step 3 is directed to a reaction for obtaining a hydrobenzo[b]thiophene compound (XXVIIa) in which a double bond between the 2- and 3-positions of the thiophene ring is reduced, by reducing the benzo[b]thiophene compound (XXXV) obtained in step 2.

The method of the above reduction is not specially limited, while a method of the reduction with hydrogen in the presence of a catalyst such as palladium or platinum oxide under atmospheric pressure or elevated pressure is facile and preferred.

After the completion of the reaction, according to a conventional method, the catalyst is removed, and the solvent is distilled off, whereby the intended hydrobenzo[b]thiophene compound (XXVIIa) can be isolated.

A halogenation, a Grignard reaction and an oxidation thereafter can be accomplished in the same manner as in the steps 3, 4 and 5 in Production scheme 7, whereby the intended aromatic carboxylic acid derivative (IIy) can be obtained.

The aromatic carboxylic acid derivative of the general formula (IIy) can be generally produced by the method in the following Production scheme 10.

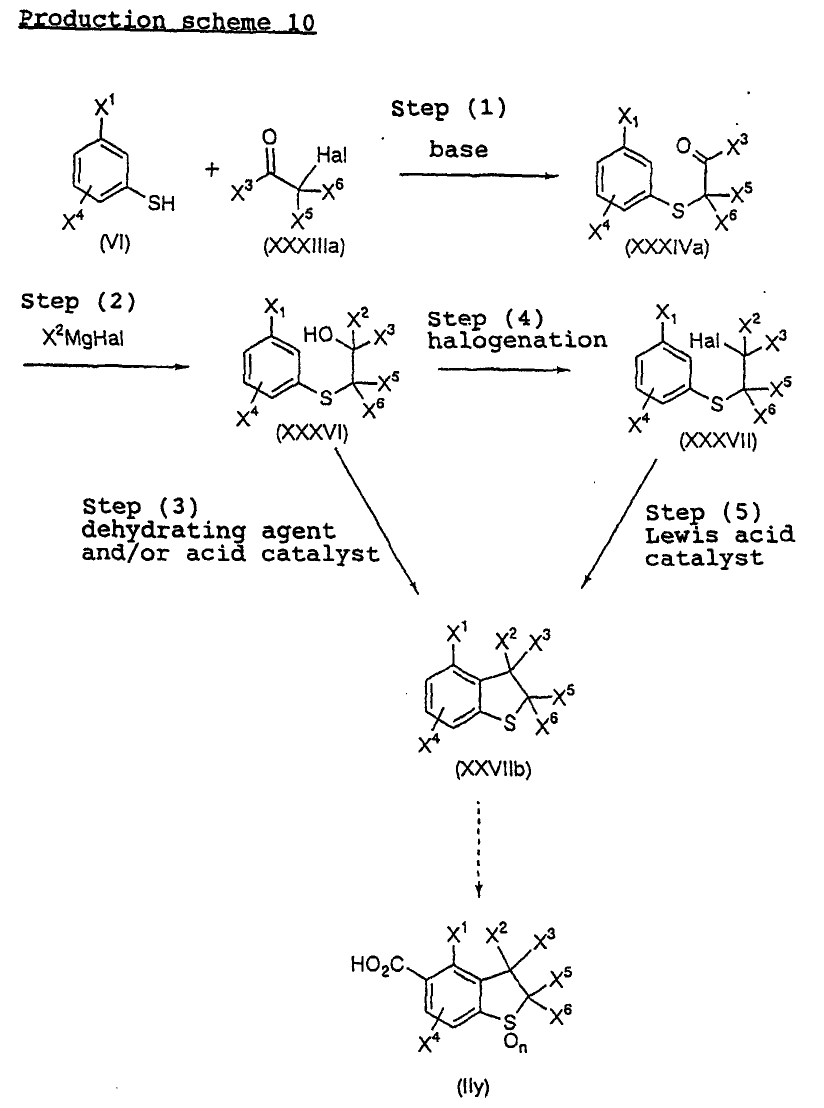

### (Step 1)

A thiophenol of the formula (VI) and a ketone of the formula (XXXIIIa) are reacted with each other in the same manner as in the above Production scheme 9, to produce a sulfide of the formula (XXXIVa).

### (Step 2)

This is a step in which a Grignard reagent is reacted with the sulfide of the formula (XXXIVa) obtained in step 1, to form an alcohol of the formula (XXXVI). The reaction in this step is a typical Grignard reaction, and its details are therefore omitted.

Steps 3, 4 and 5 thereafter can be carried out in the same manner as in the steps 3, 4 and 5 in Production scheme 4, and their details are therefore omitted. The procedures thereafter are carried out in the same manner as in the above Production scheme 7, to obtain the aromatic carboxylic acid derivative of the formula (IIy).

The aromatic carboxylic acid derivative of the general formula (II) in which X⁴ is hydrogen can be produced by the following Production scheme 11.

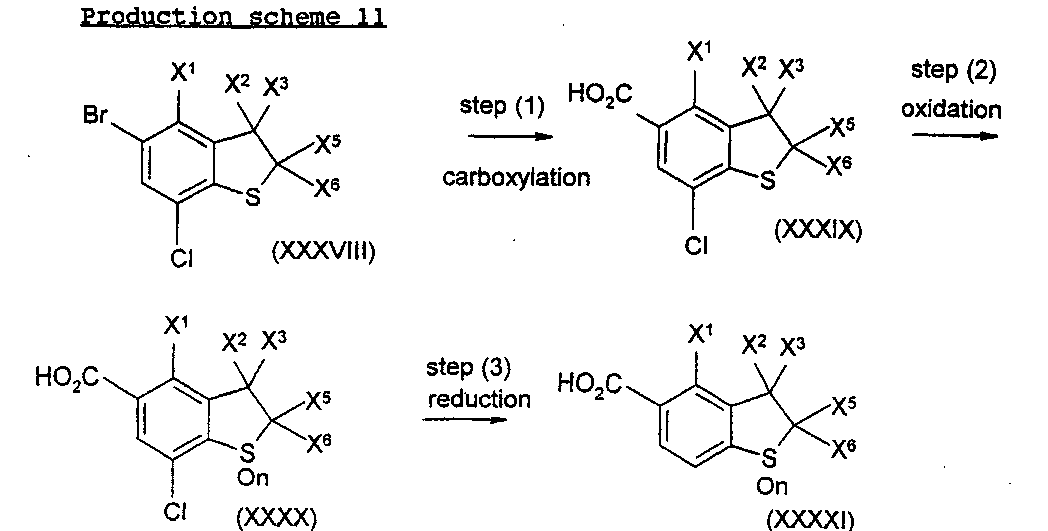

### (Steps 1 and 2)

These are steps in which the starting material of the formula (XXXVIII) obtained in the steps 1 ∼ 3 in Production scheme 1 or in the steps 1 ∼ 3 in Production scheme 4 or 7 are treated in the same manner as in the steps 4 and 5 in Production scheme 1 through an aromatic carboxylic acid derivative of the formula (XXXIX) (n = 0, sulfide compound), to form an aromatic carboxylic acid derivative of the formula (XXXX) (n = 1, sulfone compound/n = 2, sulfoxide compound). For details of these reactions, see the explanation of Production scheme 1.

### (Step 3)

This is a step in which the compound of the formula (XXXX) is reduced to form an aromatic carboxylic acid derivative of the formula (XXXXI) (n = 1 or 2). The reduction method is not specially limited. For example, there is a method in which the reduction is carried out with hydrogen having atmospheric pressure or elevated pressure in the presence of a catalyst such as palladium or platinum oxide, or it is directly carried out in the presence of zinc powder without using a catalyst. For accepting hydrogen chloride formed in the reaction, a base such as triethylamine, pyridine, sodium hydroxide or potassium hydroxide is allowed to be co-present in an amount equal to or greater than the equivalent weight based on the compound of the formula (XXXX). As a solvent, it is preferred to use an alcohol solvent such as methanol or ethanol. For fully dissolving the starting material, it is preferred to use ethanol having a water content of about 60 %. The reaction temperature is generally 20 ∼ 120°C, and the reaction time is generally approximately 1 ∼ 12 hours.

An aromatic carboxylic acid derivative of the formula (II-A) X² and X⁵ together form a bond, i.e., a compound of the formula (IIzb), is produced by the following Production scheme 14.

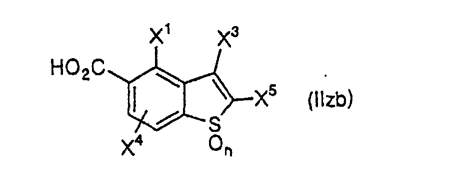

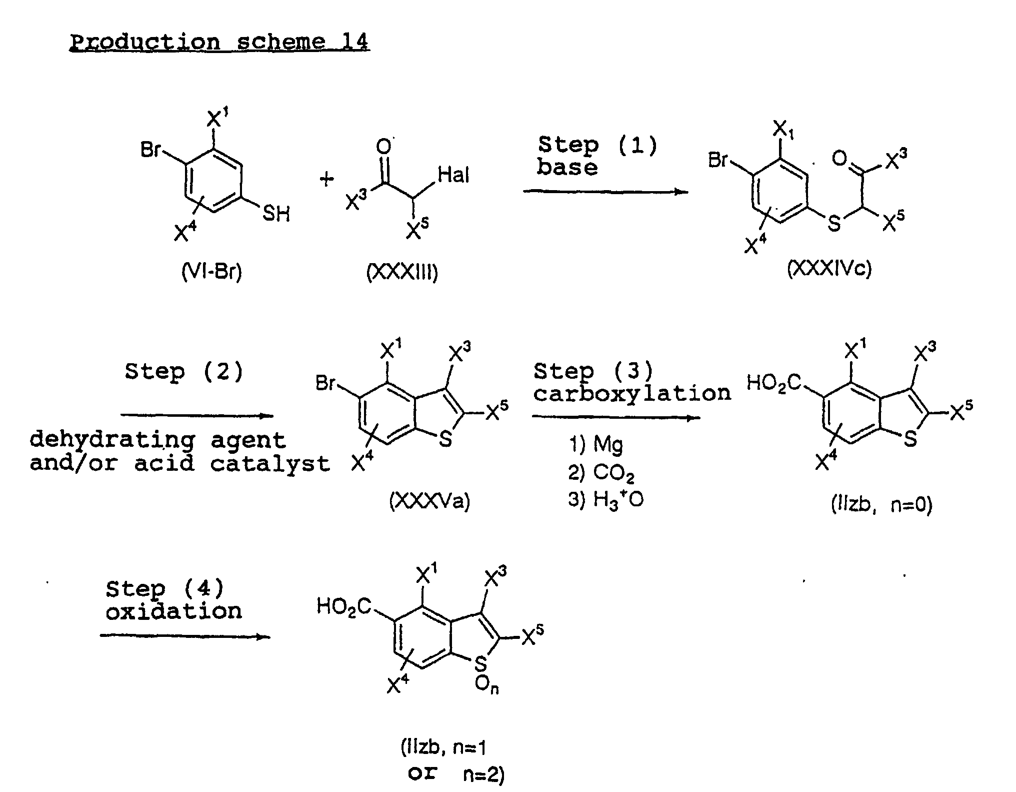

As a starting material, a bromine-substituted thiophenol derivative of the formula (VI-Br) is used in place of the substituted thiophenol of the formula (VI) in Production scheme 9. The bromine-substituted thiophenol derivative is also obtained by a known method similar to the method of producing the substituted thiophenol.

The subsequent steps 1 and 2 are essentially the same as the steps 1 and 2 in Production scheme 9, and the steps 3 and 4 are the same as the step 4 or 5 in Production scheme 7. Their details are therefore omitted.

The 5-hydroxypyrazole of the general formula (III) as a starting material for the production of the pyrazole derivative (I-A) of the present invention can be produced by one of the following methods depending upon its substituent. In the following reaction schemes, R¹ and R² are as defined in the general formula (I-A).
(1) Method disclosed in East Germany Patent 83145 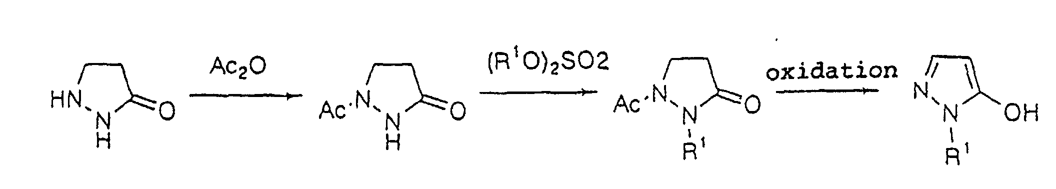
(2) Method disclosed in U. S. Patent 4,744,815 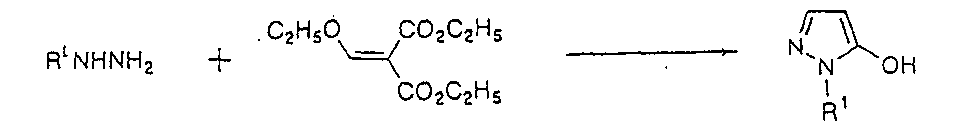
(3) Method disclosed in JP-A-3-44375

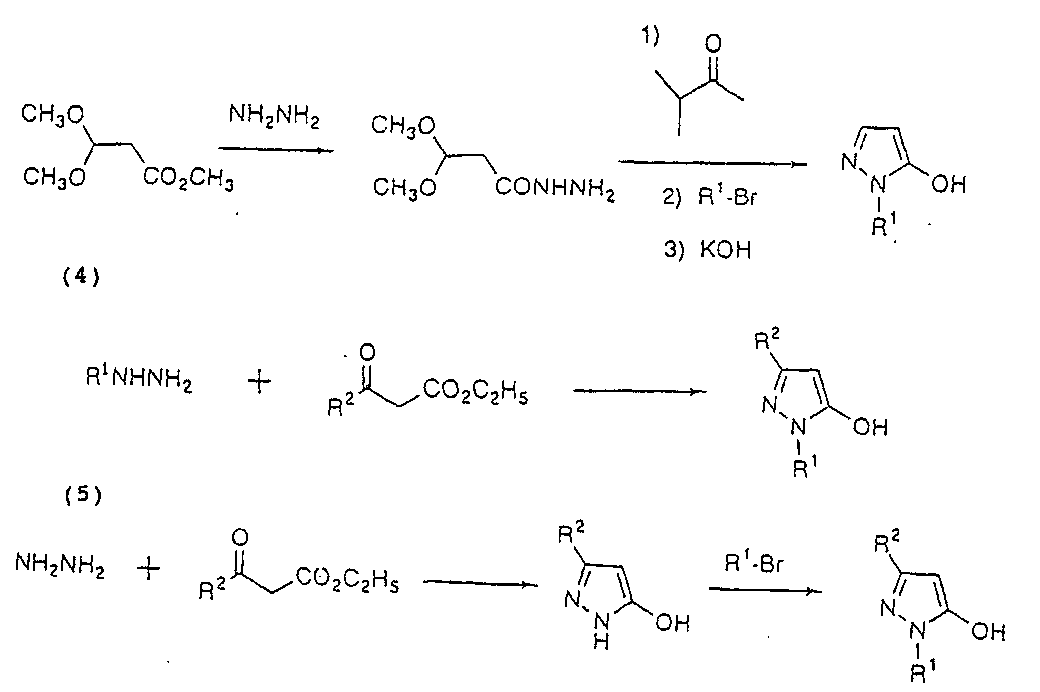

The above (1) ∼ (3) show a method of producing 5-hydroxypyrazole of the general formula (III) in which R² = hydrogen atom.

The above (4) and (5) show a method of producing 5-hydroxypyrazole of the general formula (III) in which R² = C₁∼C₄ alkyl group, C₁∼C₄ haloalkyl group or C₂∼C₄ alkoxyalkyl group.

The following Table 1 shows examples of compounds of the present invention which are pyrazole derivatives of the general formula (I) in which X⁵ = X⁶ = hydrogen atom.

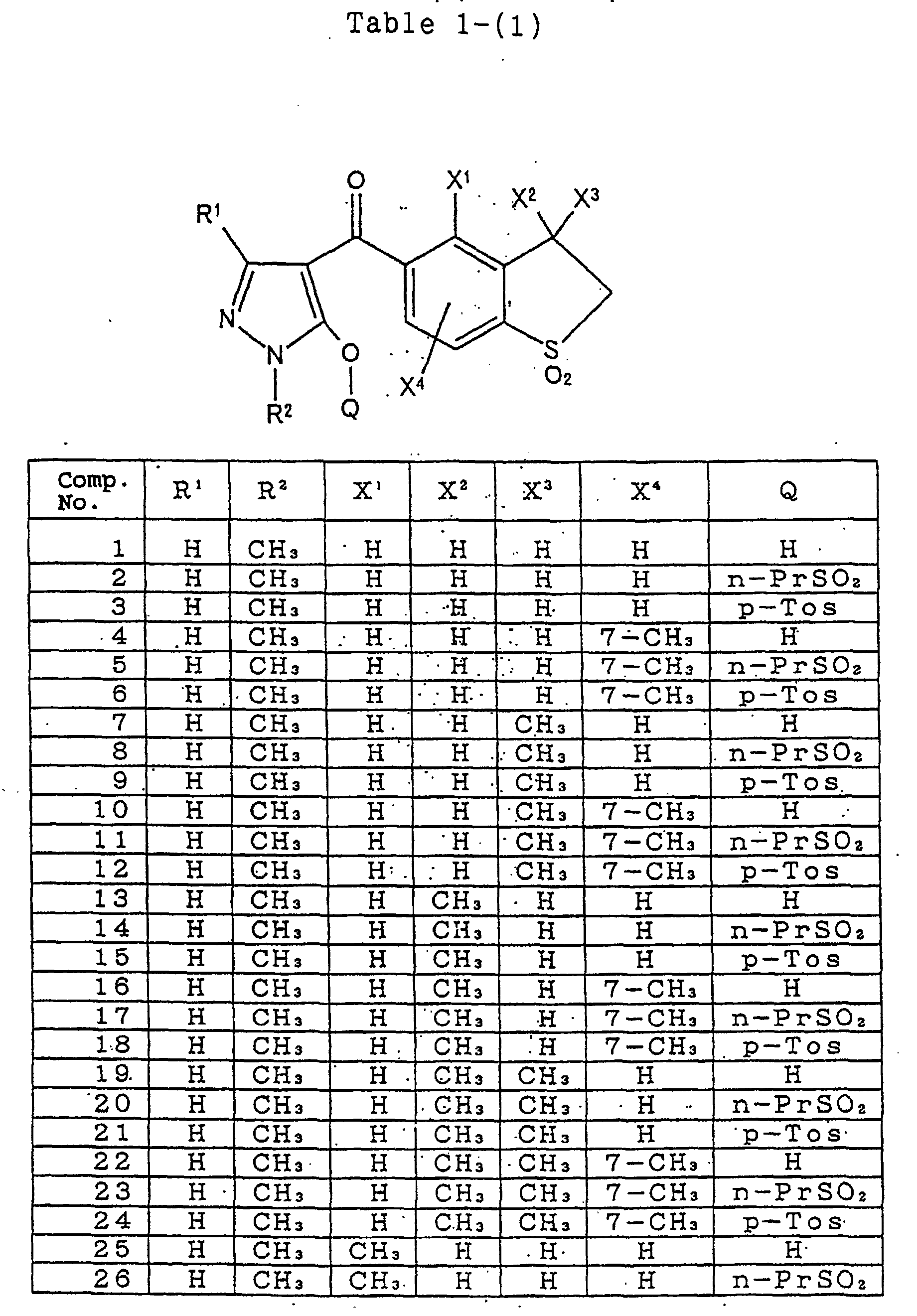

**Table 1-(2)**

| Comp No. | R¹ | R² | X¹ | X² | X³ | X⁴ | Q |
|---|---|---|---|---|---|---|---|
| 27 | H | CH₃ | CH₃ | H | H | H | p-Tos |
| 28 | H | CH₃ | CH₃ | H | H | 7-CH₃ | H |
| 29 | H | CH₃ | CH₃ | H | H | 7-CH₃ | n-PrSO₂ |
| 30 | H | CH₃ | CH₃ | H | H | 7-CH₃ | p-Tos |
| 31 | H | CH₃ | CH₃ | H | CH₃ | H | H |
| 32 | H | CH₃ | CH₃ | H | CH₃ | H | n-PrSO₂ |
| 33 | H | CH₃ | CH₃ | H | CH₃ | H | p-Tos |
| 34 | H | CH₃ | CH₃ | H | CH₃ | 7-CH₃ | H |
| 35 | H | CH₃ | CH₃ | H | CH₃ | 7-CH₃ | n-PrSO₂ |
| 36 | H | CH₃ | CH₃ | H | CH₃ | 7-CH₃ | p-Tos |
| 37 | H | CH₃ | CH₃ | CH₃ | H | H | H |
| 38 | H | CH₃ | CH₃ | CH₃ | H | H | n-PrSO₂ |
| 39 | H | CH₃ | CH₃ | CH₃ | H | H | p-Tos |
| 40 | H | CH₃ | CH₃ | CH₃ | H | 7-CH₃ | H |
| 41 | H | CH₃ | CH₃ | CH₃ | H | 7-CH₃ | n-PrSO₂ |
| 42 | H | CH₃ | CH₃ | CH₃ | H | 7-CH₃ | p-Tos |
| 43 | H | CH₃ | CH₃ | CH₃ | CH₃ | H | H |
| 44 | H | CH₃ | CH₃ | CH₃ | CH₃ | H | n-PrSO₂ |
| 45 | H | CH₃ | CH₃ | CH₃ | CH₂ | H | p-Tos |
| 46 | H | CH₃ | CH₃ | CH₃ | CH₃ | 7-CH₃ | H |
| 47 | H | CH₃ | CH₃ | CH₃ | CH₃ | 7-CH₃ | n-PrSO₂ |
| 48 | H | CH₃ | CH₃ | CH₃ | CH₃ | 7-CH₃ | p-Tos |
| 49 | H | C₂H₅ | H | H | H | H | H |
| 50 | H | C₂H₅ | H | H | H | H | n-PrSO₂ |
| 51 | H | C₂H₅ | H | H | H | H | p-Tos |
| 52 | H | C₂H₅ | H | H | H | 7-CH₃ | H |
| 53 | H | C₂H₅ | H | H | H | 7-CH₃ | n-PrSO₂ |
| 54 | H | C₂H₅ | H | H | H | 7-CH₃ | p-Tos |
| 55 | H | C₂H₅ | H | H | CH₃ | H | H |
| 56 | H | C₂H₅ | H | H | CH₃ | H | n-PrSO₂ |
| 57 | H | C₂H₅ | H | H | CH₃ | H | p-Tos |
| 58 | H | C₂H₅ | H | H | CH₃ | 7-CH₃ | H |
| 59 | H | C₂H₅ | H | H | CH₃ | 7-CH₃ | n-PrSO₂ |
| 60 | H | C₂H₅ | H | H | CH₃ | 7-CH₃ | p-Tos |
| 61 | H | C₂H₅ | H | CH₃ | H | H | H |
| 62 | H | C₂H₅ | H | CH₃ | H | H | n-PrSO₂ |
| 63 | H | C₂H₅ | H | CH₃ | H | H | p-Tos |

**Table 1-(3)**

| Comp. No. | R¹ | R² | X¹ | X² | X³ | X⁴ | Q |
|---|---|---|---|---|---|---|---|
| 64 | H | C₂H₅ | H | CH₃ | H | 7-CH₃ | H |
| 65 | H | C₂H₅ | H | CH₃ | H | 7-CH₃ | n-PrSO₂ |
| 66 | H | C₂H₅ | H | CH₃ | H | 7-CH₃ | p-Tos |
| 67 | H | C₂H₅ | H | CH₃ | CH₃ | H | H |
| 68 | H | C₂H₅ | H | CH₃ | CH₃ | H | n-PrSO₂ |
| 69 | H | C₂H₅ | H | CH₃ | CH₃ | H | p-Tos |
| 70 | H | C₂H₅ | H | CH₃ | CH₃ | 7-CH₃ | H |
| 71 | H | C₂H₅ | H | CH₃ | CH₃ | 7-CH₃ | n-PrSO₂ |
| 72 | H | C₂H₅ | H | CH₃ | CH₃ | 7-CH₃ | p-Tos |
| 73 | H | C₂H₅ | CH₃ | H | H | H | H |
| 74 | H | C₂H₅ | CH₃ | H | H | H | n-PrSO₂ |
| 75 | H | C₂H₅ | CH₃ | H | H | H | p-Tos |
| 76 | H | C₂H₅ | CH₃ | H | H | 7-CH₃ | H |
| 77 | H | C₂H₅ | CH₃ | H | H | 7-CH₃ | n-PrSO₂ |
| 78 | H | C₂H₅ | CH₃ | H | H | 7-CH₃ | p-Tos |
| 79 | H | C₂H₅ | CH₃ | H | CH₃ | H | H |
| 80 | H | C₂H₅ | CH₃ | H | CH₃ | H | n-PrSO₂ |
| 81 | H | C₂H₅ | CH₃ | H | CH₃ | H | p-Tos |
| 82 | H | C₂H₅ | CH₃ | H | CH₃ | 7-CH₃ | H |
| 83 | H | C₂H₅ | CH₃ | H | CH₃ | 7-CH₃ | n-PrSO₂ |
| 84 | H | C₂H₅ | CH₃ | H | CH₃ | 7-CH₃ | p-Tos |
| 85 | H | C₂H₅ | CH₃ | CH₃ | H | H | H |
| 86 | H | C₂H₅ | CH₃ | CH₃ | H | H | n-PrSO₂ |
| 87 | H | C₂H₅ | CH₃ | CH₃ | H | H | p-Tos |
| 88 | H | C₂H₅ | CH₃ | CH₃ | H | 7-CH₃ | H |
| 89 | H | C₂H₅ | CH₃ | CH₃ | H | 7-CH₃ | n-PrSO₂ |
| 90 | H | C₂H₅ | CH₃ | CH₃ | H | 7-CH₃ | p-Tos |
| 91 | H | C₂H₅ | CH₃ | CH₃ | CH₃ | H | H |
| 92 | H | C₂H₅ | CH₃ | CH₃ | CH₃ | H | n-PrSO₂ |
| 93 | H | C₂H₅ | CH₃ | CH₃ | CH₃ | H | p-Tos |
| 94 | H | C₂H₅ | CH₃ | CH₃ | CH₃ | 7-CH₃ | H |
| 95 | H | C₂H₅ | CH₃ | CH₃ | CH₃ | 7-CH₃ | n-PrSO₂ |
| 96 | H | C₂H₅ | CH₃ | CH₃ | CH₃ | 7-CH₃ | p-Tos |
| 97 | H | C₃H₇ | H | H | H | H | H |
| 98 | H | C₃H₇ | H | H | H | H | n-PrSO₂ |
| 99 | H | C₃H₇ | H | H | H | H | p-Tos |
| 100 | H | C₃H₇ | H | H | H | 7-CH₃ | H |

**Table 1-(4)**

| Comp. No. | R¹ | R² | X¹ | X² | X³ | X⁴ | Q |
|---|---|---|---|---|---|---|---|
| 101 | H | C₃H₇ | H | H | H | 7-CH₃ | n-PrSO₂ |
| 102 | H | C₃H₇ | H | H | H | 7-CH₃ | p-Tos |
| 103 | H | C₃H₇ | H | H | CH₃ | H | H |
| 104 | H | C₃H₇ | H | H | CH₃ | H | n-PrSO₂ |
| 105 | H | C₃H₇ | H | H | CH₃ | H | p-Tos |
| 106 | H | C₃H₇ | H | H | CH₃ | 7-CH₃ | H |
| 107 | H | C₃H₇ | H | H | CH₃ | 7-CH₃ | n-PrSO₂ |
| 108 | H | C₃H₇ | H | H | CH₃ | 7-CH₃ | p-Tos |
| 109 | H | C₃H₇ | H | CH₃ | H | H | H |
| 110 | H | C₃H₇ | H | CH₃ | H | H | n-PrSO₂ |
| 111 | H | C₃H₇ | H | CH₃ | H | H | p-Tos |
| 112 | H | C₃H₇ | H | CH₃ | H | 7-CH₃ | H |
| 113 | H | C₃H₇ | H | CH₃ | H | 7-CH₃ | n-PrSO₂ |
| 114 | H | C₃H₇ | H | CH₃ | H | 7-CH₃ | p-Tos |
| 115 | H | C₃H₇ | H | CH₃ | CH₃ | H | H |
| 116 | H | C₃H₇ | H | CH₃ | CH₃ | H | n-PrSO₂ |
| 117 | H | C₃H₇ | H | CH₃ | CH₃ | H | p-Tos |
| 118 | H | C₃H₇ | H | CH₃ | CH₃ | 7-CH₃ | H |
| 119 | H | C₃H₇ | H | CH₃ | CH₃ | 7-CH₃ | n-PrSO₂ |
| 120 | H | C₃H₇ | H | CH₃ | CH₃ | 7-CH₃ | p-Tos |
| 121 | H | C₃H₇ | CH₃ | H | H | H | H |
| 122 | H | C₃H₇ | CH₃ | H | H | H | n-PrSO₂ |
| 123 | H | C₃H₇ | CH₃ | H | H | H | p-Tos |
| 124 | H | C₃H₇ | CH₃ | H | H | 7-CH₃ | H |
| 125 | H | C₃H₇ | CH₃ | H | H | 7-CH₃ | n-PrSO₂ |
| 126 | H | C₃H₇ | CH₃ | H | H | 7-CH₃ | p-Tos |
| 127 | H | C₃H₇ | CH₃ | H | CH₃ | H | H |
| 128 | H | C₃H₇ | CH₃ | H | CH₃ | H | n-PrSO₂ |
| 129 | H | C₃H₇ | CH₃ | H | CH₃ | H | p-Tos |
| 130 | H | C₃H₇ | CH₃ | H | CH₃ | 7-CH₃ | H |
| 131 | H | C₃H₇ | CH₃ | H | CH₃ | 7-CH₃ | n-PrSO₂ |
| 132 | H | C₃H₇ | CH₃ | H | CH₃ | 7-CH₃ | p-Tos |
| 133 | H | C₃H₇ | CH₃ | CH₃ | H | H | H |
| 134 | H | C₃H₇ | CH₃ | CH₃ | H | H | n-PrSO₂ |
| 135 | H | C₃H₇ | CH₃ | CH₃ | H | H | p-Tos |
| 136 | H | C₃H₇ | CH₃ | CH₃ | H | 7-CH₃ | H |
| 137 | H | C₃H₇ | CH₃ | CH₃ | H | 7-CH₃ | n-PrSO₂ |

**Table 1-(5)**

| Comp. No. | R¹ | R² | X¹ | X² | X³ | X⁴ | Q |
|---|---|---|---|---|---|---|---|
| 138 | H | C₃H₇ | CH₃ | CH₃ | H | 7-CH₃ | p-Tos |
| 139 | H | C₃H₇ | CH₃ | CH₃ | CH₃ | H | H |
| 140 | H | C₃H₇ | CH₃ | CH₃ | CH₃ | H | n-PrSO₂ |
| 141 | H | C₃H₇ | CH₃ | CH₃ | CH₃ | H | p-Tos |
| 142 | H | C₃H₇ | CH₃ | CH₃ | CH₃ | 7-CH₃ | H |
| 143 | H | C₃H₇ | CH₃ | CH₃ | CH₃ | 7-CH₃ | n-PrSO₂ |
| 144 | H | C₃H₇ | CH₃ | CH₃ | CH₃ | 7-CH₃ | p-Tos |
| 145 | CH₃ | CH₃ | H | H | H | H | H |
| 146 | CH₃ | C₃H₇ | H | H | H | H | n-PrSO₂ |
| 147 | CH₃ | C₃H₇ | H | H | H | H | p-Tos |
| 148 | CH₃ | C₃H₇ | H | H | H | 7-CH₃ | H |
| 149 | CH₃ | C₃H₇ | H | H | H | 7-CH₃ | n-PrSO₂ |
| 150 | CH₃ | C₃H₇ | H | H | H | 7-CH₃ | p-Tos |
| 151 | CH₃ | C₃H₇ | H | H | CH₃ | H | H |
| 152 | CH₃ | C₃H₇ | H | H | CH₃ | H | n-PrSO₂ |
| 153 | CH₃ | C₃H₇ | H | H | CH₃ | H | p-Tos |
| 154 | CH₃ | C₃H₇ | H | H | CH₃ | 7-CH₃ | H |
| 155 | CH₃ | C₃H₇ | H | H | CH₃ | 7-CH₃ | n-PrSO₂ |
| 156 | CH₃ | C₃H₇ | H | H | CH₃ | 7-CH₃ | p-Tos |
| 157 | CH₃ | C₃H₇ | H | CH₃ | H | H | H |
| 158 | CH₃ | C₃H₇ | H | CH₃ | H | H | n-PrSO₂ |
| 159 | CH₃ | C₃H₇ | H | CH₃ | H | H | p-Tos |
| 160 | CH₃ | C₃H₇ | H | CH₃ | H | 7-CH₃ | H |
| 161 | CH₃ | C₃H₇ | H | CH₃ | H | 7-CH₃ | n-PrSO₂ |
| 162 | CH₃ | C₃H₇ | H | CH₃ | H | 7-CH₃ | p-Tos |
| 163 | CH₃ | C₃H₇ | H | CH₃ | CH₃ | H | H |
| 164 | CH₃ | C₃H₇ | H | CH₃ | CH₃ | H | n-PrSO₂ |
| 165 | CH₃ | C₃H₇ | H | CH₃ | CH₃ | H | p-Tos |
| 166 | CH₃ | C₃H₇ | H | CH₃ | CH₃ | 7-CH₃ | H |
| 167 | CH₃ | C₃H₇ | H | CH₃ | CH₃ | 7-CH₃ | n-PrSO₂ |
| 168 | CH₃ | C₃H₇ | H | CH₃ | CH₃ | 7-CH₃ | p-Tos |
| 169 | CH₃ | C₃H₇ | CH₃ | H | H | H | H |
| 170 | CH₃ | C₃H₇ | CH₃ | H | H | H | n-PrSO₂ |
| 171 | CH₃ | C₃H₇ | CH₃ | H | H | H | p-Tos |
| 172 | CH₃ | C₃H₇ | CH₃ | H | H | 7-CH₃ | H |
| 173 | CH₃ | C₃H₇ | CH₃ | H | H | 7-CH₃ | n-PrSO₂ |
| 174 | CH₃ | C₃H₇ | CH₃ | H | H | 7-CH₃ | p-Tos |

**Table 1-(6)**

| Comp. No. | R¹ | R² | X¹ | X² | X³ | X⁴ | Q |
|---|---|---|---|---|---|---|---|
| 175 | CH₃ | C₃H₇ | CH₃ | H | CH₃ | H | H |
| 176 | CH₃ | C₃H₇ | CH₃ | H | CH₃ | H | n-PrSO₂ |
| 177 | CH₃ | C₃H₇ | CH₃ | H | CH₃ | H | p-Tos |
| 178 | CH₃ | C₃H₇ | CH₃ | H | CH₃ | 7-CH₃ | H |
| 179 | CH₃ | C₃H₇ | CH₃ | H | CH₃ | 7-CH₃ | n-PrSO₂ |
| 180 | CH₃ | C₃H₇ | CH₃ | H | CH₃ | 7-CH₃ | p-Tos |
| 181 | CH₃ | C₃H₇ | CH₃ | CH₃ | H | H | H |
| 182 | CH₃ | C₃H₇ | CH₃ | CH₃ | H | H | n-PrSO₂ |
| 183 | CH₃ | C₃H₇ | CH₃ | CH₃ | H | H | p-Tos |
| 184 | CH₃ | C₃H₇ | CH₃ | CH₃ | H | 7-CH₃ | H |
| 185 | CH₃ | C₃H₇ | CH₃ | CH₃ | H | 7-CH₃ | n-PrSO₂ |
| 186 | CH₃ | C₃H₇ | CH₃ | CH₃ | H | 7-CH₃ | p-Tos |
| 187 | CH₃ | C₃H₇ | CH₃ | CH₃ | CH₃ | H | H |
| 188 | CH₃ | C₃H₇ | CH₃ | CH₃ | CH₃ | H | n-PrSO₂ |
| 189 | CH₃ | C₃H₇ | CH₃ | CH₃ | CH₃ | H | p-Tos |
| 190 | CH₃ | C₃H₇ | CH₃ | CH₃ | CH₃ | 7-CH₃ | H |
| 191 | CH₃ | C₃H₇ | CH₃ | CH₃ | CH₃ | 7-CH₃ | n-PrSO₂ |
| 192 | CH₃ | C₃H₇ | CH₃ | CH₃ | CH₃ | 7-CH₃ | p-Tos |
| 193 | CH₃ | C₂H₅ | H | H | H | H | H |
| 194 | CH₃ | C₂H₅ | H | H | H | H | n-PrSO₂ |
| 195 | CH3 | C₂H₅ | H | H | H | H | p-Tos |
| 196 | CH₃ | C₂H₅ | H | H | H | 7-CH₃ | H |
| 197 | CH₃ | C₂H₅ | H | H | H | 7-CH₃ | n-PrSO₂ |
| 198 | CH₃ | C₂H₅ | H | H | H | 7-CH₃ | p-Tos |
| 199 | CH₃ | C₂H₅ | H | H | CH₃ | H | H |
| 200 | CH₃ | C₂H₅ | H | H | CH₃ | H | n-PrSO₂ |
| 201 | CH₃ | C₂H₅ | H | H | CH₃ | H | p-Tos |
| 202 | CH₃ | C₂H₅ | H | H | CH₃ | 7-CH₃ | H |
| 203 | CH₃ | C₂H₅ | H | -H. | CH₃ | 7-CH₃ | n-PrSO₂ |
| 204 | CH₃ | C₂H₅ | H: | H | CH₃ | 7-CH₃ | p-Tos |
| 205 | CH₃ | C₂H₅ | H | CH₃ | H | H | H |
| 206 | CH₃ | C₂H₅ | H | CH₃ | H | H | n-PrSO₂ |
| 207 | CH₃ | C₂H₅ | H | CH₃ | H | H | p-Tos |
| 208 | CH₃ | C₂H₅ | H | CH₃ | H | 7-CH₃ | H |
| 209 | CH₃ | C₂H₅ | H | CH₃ | H | 7-CH₃ | n-PrSO₂ |
| 210 | CH₃ | C₂H₅ | H | CH₃ | H | 7-CH₃ | p-Tos |
| 211 | CH₃ | C₂H₅ | H | CH₃ | CH₃ | H | H |

**Table 1-(7)**

| Comp. No. | R¹ | R² | X¹ | X² | X³ | X⁴ | Q |
|---|---|---|---|---|---|---|---|
| 212 | CH₃ | C₂H₅ | H | CH₃ | CH₃ | H | n-PrSO₂ |
| 213 | CH₃ | C₂H₅ | H | CH₃ | CH₃ | H | p-Tos |
| 214 | CH₃ | C₂H₅ | H | CH₃ | CH₃ | 7-CH₃ | H |
| 215 | CH₃ | C₂H₅ | H | CH₃ | CH₃ | 7-CH₃ | n-PrSO₂ |
| 216 | CH₃ | C₂H₅ | H | CH₃ | CH₃ | 7-CH₃ | p-Tos |
| 217 | CH₃ | C₂H₅ | CH₃ | H | H | H | H |
| 218 | CH₃ | C₂H₅ | CH₃ | H | H | H | n-PrSO₂ |
| 219 | CH₃ | C₂H₅ | CH₃ | H | H | H | p-Tos |
| 220 | CH₃ | C₂H₅ | CH₃ | H | H | 7-CH₃ | H |
| 221 | CH₃ | C₂H₅ | CH₃ | H | H | 7-CH₃ | n-PrSO₂ |
| 222 | CH₃ | C₂H₅ | CH₃ | H | H | 7-CH₃ | p-Tos |
| 223 | CH₃ | C₂H₅ | CH₃ | H | CH₃ | H | H |
| 224 | CH₃ | C₂H₅ | CH₃ | H | CH₃ | H | n-PrSO₂ |
| 225 | CH₃ | C₂H₅ | CH₃ | H | CH₃ | H | p-Tos |
| 226 | CH₃ | C₂H₅ | CH₃ | H | CH₃ | 7-CH₃ | H |
| 227 | CH₃ | C₂H₅ | CH₃ | H | CH₃ | 7-CH₃ | n-PrSO₂ |
| 228 | CH₃ | C₂H₅ | CH₃ | H | CH₃ | 7-CH₃ | p-Tos |
| 229 | CH₃ | C₂H₅ | CH₃ | CH₃ | H | H | H |
| 230 | CH₃ | C₂H₅ | CH₃ | CH₃ | H | H | n-PrSO₂ |
| 231 | CH₃ | C₂H₅ | CH₃ | CH₃ | H | H | p-Tos |
| 232 | CH₃ | C₂H₅ | CH₃ | CH₃ | H | 7-CH₃ | H |
| 233 | CH₃ | C₂H₅ | CH₃ | CH₃ | H | 7-CH₃ | n-PrSO₂ |
| 234 | CH₃ | C₂H₅ | CH₃ | CH₃ | H | 7-CH₃ | p-Tos |
| 235 | CH₃ | C₂H₅ | CH₃ | CH₃ | CH₃ | H | H |
| 236 | CH₃ | C₂H₅ | CH₃ | CH₃ | CH₃ | H | n-PrSO₂ |
| 237 | CH₃ | C₂H₅ | CH₃ | CH₃ | CH₃ | H | p-Tos |
| 238 | CH₃ | C₂H₅ | CH₃ | CH₃ | CH₃ | 7-CH₃ | H |
| 239 | CH₃ | C₂H₅ | CH₃ | CH₃ | CH₃ | 7-CH₃ | n-PrSO₂ |
| 240 | CH₃ | C₂H₅ | CH₃ | CH₃ | CH₃ | 7-CH₃ | p-Tos |
| 241 | CH₃ | C₃H₇ | H | H | H | H | H |
| 242 | CH₃ | C₃H₇ | H | H | H | H | n-PrSO₂ |
| 243 | CH₃ | C₃H₇ | H | H | H | H | p-Tos |
| 244 | CH₃ | C₃H₇ | H | H | H | 7-CH₃ | H |
| 245 | CH₃ | C₃H₇ | H | H | H | 7-CH₃ | n-PrSO₂ |
| 246 | CH₃ | C₃H₇ | H | H | H | 7-CH₃ | p-Tos |
| 247 | CH₃ | C₃H₇ | H | H | CH₃ | H | H |
| 248 | CH₃ | C₃H₇ | H | H | CH₃ | H | n-PrSO₂ |

**Table 1-(8)**

| Comp. No. | R¹ | R² | X¹ | X² | X³ | X⁴ | Q |
|---|---|---|---|---|---|---|---|
| 249 | CH₃ | C₃H₇ | H | H | CH₃ | H | p-Tos |
| 250 | CH₃ | C₃H₇ | H | H | CH₃ | 7-CH₃ | H |
| 251 | CH₃ | C₃H₇ | H | H | CH₃ | 7-CH₃ | n-PrSO₂ |
| 252 | CH₃ | C₃H₇ | H | H | CH₃ | 7-CH₃ | p-Tos |
| 253 | CH₃ | C₃H₇ | H | CH₃ | H | H | H |
| 254 | CH₃ | C₃H₇ | H | CH₃ | H | H | n-PrSO₂ |
| 255 | CH₃ | C₃H₇ | H | CH₃ | H | H | p-Tos |
| 256 | CH₃ | C₃H₇ | H | CH₃ | H | 7-CH₃ | H |
| 257 | CH₃ | C₃H₇ | H | CH₃ | H | 7-CH₃ | n-PrSO₂ |
| 258 | CH₃ | C₃H₇ | H | CH₃ | H | 7-CH₃ | p-Tos |
| 259 | CH₃ | C₃H₇ | H | CH₃ | CH₃ | H | H |
| 260 | CH₃ | C₃H₇ | H | CH₃ | CH₃ | H | n-PrSO₂ |
| 261 | CH₃ | C₃H₇ | H | CH₃ | CH₃ | H | p-Tos |
| 262 | CH₃ | C₃H₇ | H | CH₃ | CH₃ | 7-CH₃ | H |
| 263 | CH₃ | C₃H₇ | H | CH₃ | CH₃ | 7-CH₃ | n-PrSO₂ |
| 264 | CH₃ | C₃H₇ | H | CH₃ | CH₃ | 7-CH₃ | p-Tos |
| 265 | CH₃ | C₃H₇ | CH₃ | H | H | H | H |
| 266 | CH₃ | C₃H₇ | CH₃ | H | H | H | n-PrSO₂ |
| 267 | CH₃ | C₃H₇ | CH₃ | H | H | H | p-Tos |
| 268 | CH₃ | C₃H₇ | CH₃ | H | H | 7-CH₃ | H |
| 269 | CH₃ | C₃H₇ | CH₃ | H | H | 7-CH₃ | n-PrSO₂ |
| 270 | CH₃ | C₃H₇ | CH₃ | H | H | 7-CH₃ | p-Tos |
| 271 | CH₃ | C₃H₇ | CH₃ | H | CH₃ | H | H |
| 272 | CH₃ | C₃H₇ | CH₃ | H | CH₃ | H | n-PrSO₂ |
| 273 | CH₃ | C₃H₇ | CH₃ | H | CH₃ | H | p-Tos |
| 274 | CH₃ | C₃H₇ | CH₃ | H | CH₃ | 7-CH₃ | H |
| 275 | CH₃ | C₃H₇ | CH₃ | H | CH₃ | 7-CH₃ | n-PrSO₂ |
| 276 | CH₃ | C₃H₇ | CH₃ | H | CH₃ | 7-CH₃ | p-Tos |
| 277 | CH₃ | C₃H₇ | CH₃ | CH₃ | H | H | H |
| 278 | CH₃ | C₃H₇ | CH₃ | CH₃ | H | H | n-PrSO₂ |
| 279 | CH₃ | C₃H₇ | CH₃ | CH₃ | H | H | p-Tos |
| 280 | CH₃ | C₃H₇ | CH₃ | CH₃ | H | 7-CH₃ | H |
| 281 | CH₃ | C₃H₇ | CH₃ | CH₃ | H | 7-CH₃ | n-PrSO₂ |
| 282 | CH₃ | C₃H₇ | CH₃ | CH₃ | H | 7-CH₃ | p-Tos |
| 283 | CH₃ | C₃H₇ | CH₃ | CH₃ | CH₃ | H | H |
| 284 | CH₃ | C₃H₇ | CH₃ | CH₃ | CH₃ | H | n-PrSO₂ |
| 285 | CH₃ | C₃H₇ | CH₃ | CH₃ | CH₃ | H | p-Tos |
| 286 | CH₃ | C₃H₇ | CH₃ | CH₃ | CH₃ | 7-CH₃ | H |
| 287 | CH₃ | C₃H₇ | CH₃ | CH₃ | CH₃ | 7-CH₃ | n-PrSO₂ |
| 288 | CH₃ | C₃H₇ | CH₃ | CH₃ | CH₃ | 7-CH₃ | p-Tos |

### Examples

The present invention will be explained further in detail with reference to Examples hereinafter.

### Intermediate Preparation Example 1

3,3,4,7-Tetramethyl-2-hydrobenzo[b]thiophene-5-carboxylic acid-1,1-dioxide used as a starting material in Preparation Example 1 to be described later was prepared in the following steps.

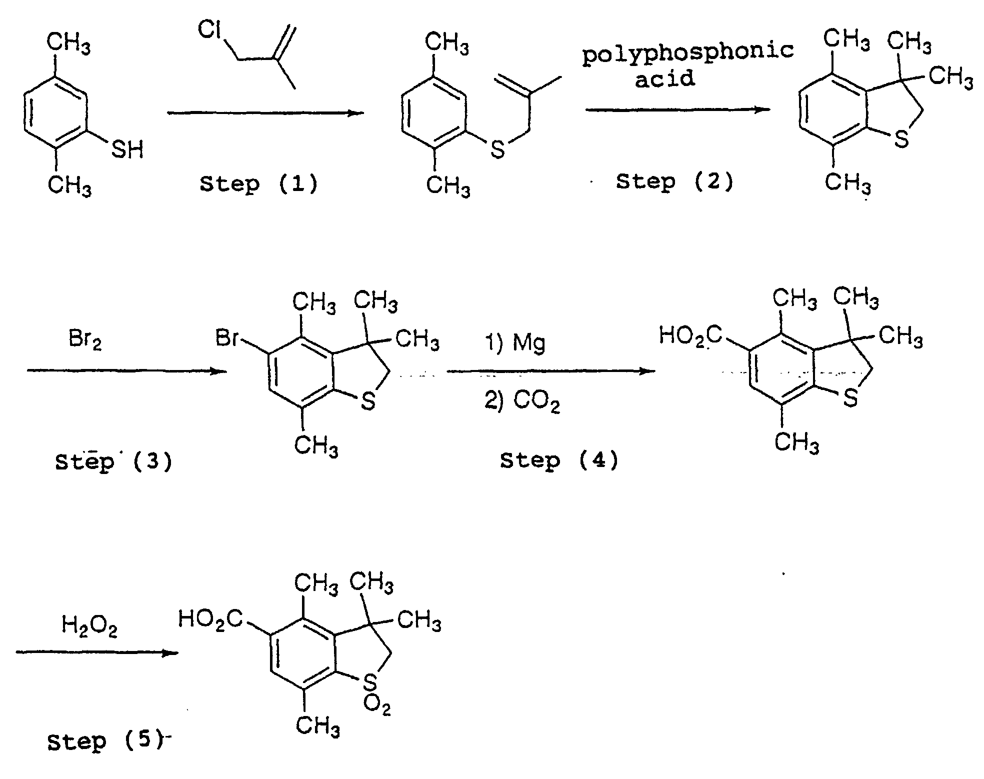

### Step (1)

A 100-ml eggplant type flask was charged with 6.9 g (50 mmol) of 2,5-dimethylthiophenol, 5.5 g (60 mmol, 1.2 equivalents) of methallyl chloride which was a halogenated olefin, 6.9 g (50 mmol, 1 equivalent) of potassium carbonate and 30 ml of acetone, and the mixture was refluxed under heat for 1 hour. The reaction mixture was allowed to cool, insolubles were removed by filtration, and the acetone was distilled off. The resultant residue was re-dispersed in n-hexane and washed with a saturated sodium chloride aqueous solution. An organic layer was dried over anhydrous sodium sulfate, and then the solvent was distilled off to give 8.6 g (yield 89 %) of 2-methyl-3-(2,5-dimethylphenylthio)-propene.
N.M.R. (ppm, solvent: deutero chloroform, internal standard: tetramethylsilane): 1.87(3H,s), 2.28(3H,s), 2.33(3H,s), 3.49(2H,s), 4.87(2H,m), 6.8-7.3(3H,m)
I.R. (KBr tablet, cm⁻¹) : 3090, 2980, 1610

### Step (2)

A 100-ml eggplant type flask was charged with 8.6 g (45 mmol) of the 2-methyl-3-(2,5-dimethylphenylthio)-1-propene obtained in the above step (1) and 50 g of polyphosphoric acid (containing 300 mmol (6.7 equivalents) of diphosphorus pentoxide (P₂O₅)) as a dehydration-condensation agent, and the mixture was allowed to react at 150°C for 2 hours. After the completion of the reaction, the reaction mixture was poured into ice water and extracted with n-hexane. The resultant organic layer was washed with a saturated sodium chloride aqueous solution and dried over anhydrous sodium sulfate. The solvent was distilled off to give a crude product, and the crude product was purified by column chromatography (elution solvent: n-hexane) to give 1.6 g (yield 19 %) of 3,3,4,7-tetramethyl-2-hydrobenzo[b]thiophene.
N.M.R. (ppm, solvent: deutero chloroform, internal standard: tetramethylsilane): 1.45(6H,s), 2.21(3H,s), 2.36(3H,s), 3.08(2H,s), 6.80(2H,dd)
I.R. (KBr tablet, cm⁻¹) : 2970, 1465, 800

### Step (3)

A 100-ml eggplant type flask was charged with 1.6 g (8 mmol) of the 3,3,4,7-tetramethyl-2-hydrobenzo[b]thiophene obtained in the above step (2) and 30 ml of chloroform, and 0.55 ml (10.7 mmol, 1.34 equivalents) of bromine was dropwise added. The mixture was allowed to react at room temperature for 1 hour, and the reaction mixture was consecutively washed with a sodium hydrogensulfite and with a saturated sodium chloride aqueous solution and dried over anhydrous sodium sulfate. The solvent was distilled off to give 1.9 g (yield 85 %) of 5-bromo-3,3,4,7-tetramethyl-2-hydrobenzo[b]thiophene.
N.M.R. (ppm, solvent: deutero chloroform, internal standard: tetramethylsilane): 1.48(6H,s), 2.18(3H,s), 2.41(3H,s), 3.08(2H,s), 7.20(H,s)
I.R. (KBr tablet, cm⁻¹) : 2950, 1440, 1100

### Step (4)

A 100-ml three-necked flask was charged with 30 ml of THF and 0.7 g (24 mmol, 3.4 equivalents) of magnesium, and 1.52 g (14 mmol, 2 equivalents) of ethyl bromide was dropwise added to activate the mixture. Then, a solution of 1.9 g (7.0 mmol) of the 5-bromo-3,3,4,7-tetramethyl-2-hydrobenzo[b]thiophene obtained in the above step (3) in 5 ml of THF was dropwise added, and the mixture was refluxed under heat for 4 hours. The reaction mixture was allowed to cool to room temperature, and carbon dioxide gas was bubbled for 2 hours. The reaction was terminated by adding 5 % hydrochloric acid to the reaction mixture, and the reaction mixture was extracted with ethyl acetate. The resultant organic layer was extracted with a potassium carbonate aqueous solution, and the resultant aqueous layer was washed with ethyl acetate, then neutralized with 5 % hydrochloric acid and extracted with ethyl acetate. The resultant organic layer was washed with a saturated sodium chloride aqueous solution and then dried over anhydrous sodium sulfate, and the solvent was distilled off to give 1.2 g (yield 70 %) of 3,3,4,7-tetramethyl-2-hydrobenzo[b]thiophene-5-carboxylic acid.
N.M.R. (ppm, solvent: deutero chloroform, internal standard: tetramethylsilane): 1.51(6H,s), 2.22(3H,s), 2.58(3H,s), 3.12(2H,s), 7.62(H,s)
I.R. (KBr tablet, cm⁻¹) : 3500, 3000, 1690

### Step (5)

A 100-ml eggplant type flask was charged with 1.2 g (4.9 mmol) of the 3,3,4,7-tetramethyl-2-hydrobenzo[b]thiophene-5-carboxylic acid, 1.7 ml (15.0 mmol, 3.1 equivalents) of 30 % H₂O₂ and 10 ml of acetic acid, and the mixture was allowed to react at 100°C for 2 hours. The reaction mixture was poured into a sodium hydrogensulfite and extracted with ethyl acetate. The resultant organic layer was washed with a saturated sodium chloride aqueous solution and dried over anhydrous sodium sulfate, and the solvent was distilled off to give 1.0 g (yield 79 %) of 3,3,4,7-tetramethyl-2-hydrobenzo[b]thiophene-5-carboxylic acid-1,1-dioxide.
N.M.R. (ppm, solvent: deutero chloroform, internal standard: tetramethylsilane): 1.69(6H,s), 2.63(6H,s), 3.38(2H,s), 7.30(H,s), 7.72(H,s)
I.R. (KBr tablet, cm⁻¹) : 3450, 1740

### Intermediate Preparation Example 2

3,4,7-Trimethyl-2-hydrobenzo[b]thiophene-5-carboxylic acid-1,1-dioxide used as a starting material in Preparation Example 2 to be described later was prepared in the following steps.

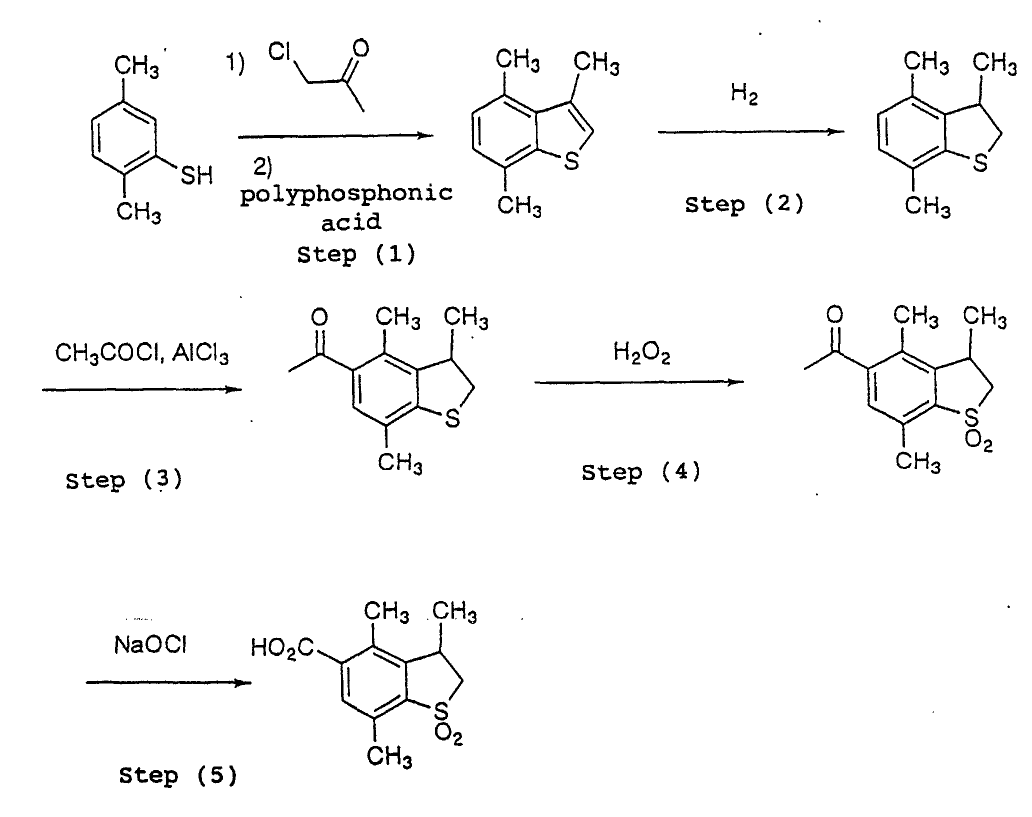

### Step (1)

A 100-ml eggplant type flask was charged with 4.0 g (29 mmol) of 2,5-dimethylthiophenol as a substituted thiophenol, 3.2 g (35 mmol, 1.2 equivalents) of chloroacetone as an α-halo-carbonyl compound, 4.0 g (29 mmol, 1 equivalent) of anhydrous potassium carbonate and 30 ml of acetone, and the mixture was refluxed under heat for 2 hours. The reaction mixture was allowed to cool, then, insolubles were removed by filtration, and the acetone was distilled off. The resultant residue was re-dispersed in n-hexane and washed with a saturated sodium chloride aqueous solution. An organic layer was dried over anhydrous sodium sulfate, and then the solvent was distilled off. To the resultant residue was added 100 g of polyphosphoric acid as a dehydration-condensation agent, and the mixture was stirred at room temperature for 1 hour. The reaction mixture was poured into ice water, and a white crystal which precipitated was re-dispersed in n-hexane and washed with a saturated sodium chloride aqueous solution. The solvent was distilled off to give 4.5 g (yield 88 %) of 3,4,7-trimethylthiophene.
N.M.R. (ppm, solvent: deutero chloroform, internal standard: tetramethylsilane): 2.48(3H,s), 2.62(3H,s), 2.72(3H,s), 2.98(3H,s)

### Step (2)

A 50-ml eggplant type flask was charged with 1.0 g of the 3,4,7-trimethylbenzo[b]thiophene obtained in the above step (1), 30 ml of ethanol and 50 mg of platinum oxide, to carry out hydrogenation under atmospheric pressure. After the completion of the reaction, the ethanol was distilled off to give 0.94 g (yield 93 %) of 3,4,7-trimethyl-2-hydrobenzo[b]thiophene.
N.M.R. (ppm, solvent: deutero chloroform, internal standard: tetramethylsilane): 1.24(3H,d), 2.21(3H,s), 2.26(3H,s), 2.7 ∼ 3.1(H,m), 3.4-3.8(2H,m), 6.98(3H,s)

### Step (3)

A 100-ml eggplant type flask was charged with 2.33 g (16.0 mmol, 1.2 equivalents) of aluminum chloride and 10 ml of dichloromethane, and the mixture was cooled with ice. To the mixture was dropwise added 1.15 ml (1.26 g, 17.5 mmol, 1.1 equivalents) of acetyl chloride, and the mixture was stirred under cooling with ice for 15 minutes. Then, a solution of 2.60 g (14.6 mmol) of 3,4,7-trimethyl-2-hydrobenzo[b]thiophene in 10 ml of dichloromethane was dropwise added.

The mixture was stirred with cooling with ice for 30 minutes, stirred at room temperature for 3 hours, and poured into ice water to terminate the reaction. An aqueous layer was extracted with dichloromethane, and an organic layer was washed with a saturated sodium chloride aqueous solution and then dried over anhydrous sodium sulfate. The solvent was distilled off to give 2.48 g (yield 77 %) of 5-acetyl-3,4,7-trimethyl-2-hydrobenzo[b]thiophene.
N.M.R. (ppm, solvent: deutero chloroform, internal standard: tetramethylsilane): 1.24(3H,d), 2.25(3H,s), 2.43(3H,s), 2.54(3H,s), 2.95(H,d), 3.5 ∼ 3.8(2H,m), 7.30(H,s)

### Step (4)

A 30-ml eggplant type flask was charged with 2.48 g (11.3 mmol) of the 5-acetyl-3,4,7-trimethyl-2-hydrobenzo[b]thiophene, 3.8 ml of a 30 % hydrogen peroxide aqueous solution and 3 ml of acetic acid, and the mixture was allowed to react at 100°C for 2 hours. The reaction mixture was poured into a sodium sulfite aqueous solution and extracted with ethyl acetate. The extract was washed with a saturated sodium chloride aqueous solution and dried over sodium sulfate, and then the solvent was distilled off to give 2.67 g (yield 94 %) of 5-acetyl-3,4,7-trimethyl-2-hydrobenzo[b]-thiophene-1,1-dioxide.
N.M.R. (ppm, solvent: deutero chloroform, internal standard: tetramethylsilane): 1.50(3H,d),-2.39(3H,s), 2.57(3H,s), 2.63(3H,s), 3.25(H,d), 3.5∼3.75(2H,m), 7.35(H,s)

### Step (5)

A 50-ml eggplant type flask was charged with 11.6 ml of 6.3 % hypochlorous acid and the content was cooled with ice. A solution of 2.67 g (10.6 mmol) of 5-acetyl-3,4,7-trimethyl-2-hydrobenzo[b]thiophene-1,1-dioxide in 10 ml of 1,4-dioxane was dropwise added. After the addition, the mixture was temperature-increased up to room temperature and then stirred for 3 hours. Then, a sodium sulfite aqueous solution was added, the reaction mixture was washed with methylene chloride twice, and then 10 ml of concentrated hydrochloric acid was added with cooling with ice. The mixture was extracted with ethyl acetate three times, and then dried over anhydrous sodium sulfate. The solvent was distilled off to give 2.38 g (yield 88 %) of 3,4,7-trimethyl-2-hydrobenzo[b]thiophene-5-carboxylic acid-1,1-dioxide.
N.M.R. (ppm, solvent: deutero chloroform, internal standard: tetramethylsilane): 1.50(3H,d), 2.58(3H,s), 2.65(3H,s), 3.28(H,d), 3.5 ∼ 3.75(2H,m), 7.85(H,s)

Preparation Examples of the novel pyrazole derivative for achieving the first object of the present invention will be explained hereinafter.

### Preparation Example 1

A 100-ml eggplant type flask was charged with 1.0 g (3.9 mmol) of the 3,3,4,7-tetramethyl-2-hydrobenzo[b]thiophene-5-carboxylic acid-1,1-dioxide, 0.48 g (4.3 mmol, 1.1 equivalents) of 1-ethyl-5-hydroxypyrazole and 100 ml of t-amyl alcohol. 1.04 g (5.0 mmol, 1.3 equivalents) of N,N'-dicyclohexylcarbodiimide (DCC) was added as a dehydrating agent, and the mixture was allowed to react at room temperature for 4 hours. To the reaction mixture was added 0.53 g (3.9 mmol, 1 equivalent) of potassium carbonate as a base, and the mixture was allowed to react further at 100°C for 2 hours. After the completion of the reaction, the solvent was distilled off, and the residue was liquid-separated by adding 50 ml of ethyl acetate and 50 ml of water. The resultant aqueous layer was neutralized with 5 % hydrochloric acid and then extracted with ethyl acetate, and the resultant organic layer was washed with a saturated sodium chloride aqueous solution and dried over anhydrous sodium sulfate. The solvent was distilled off to give 0.8 g (yield 57 %) of 3,3,4,7-tetramethyl-5-(1-ethyl-5-hydroxypyrazol-4-yl)carbonyl-2-hydrobenzo[b]thiophene-1,1-dioxide (Compound No. 94).

### Preparation Example 2

A 50-ml eggplant type flask was charged with 2.38 g (9.4 mmol) of 3,4,7-trimethyl-2-hydrobenzo[b]thiophene-5-carboxylic acid-1,1-dioxide, 1.15 g (10.3 mmol, 1.1 equivalents) of 1-ethyl-5-hydroxypyrazole and 10 ml of t-amyl alcohol, and a solution of 2.31 g (12.2 mmol, 1.3 equivalents) of DCC in 5 ml of t-amyl alcohol was added at room temperature. The mixture was allowed to react at room temperature for 2 hours, then 1.68 g (12.2 mmol, 3 equivalents) of potassium carbonate was added, and the mixture was allowed to react at 100°C for 6 hours. The solvent was distilled off, the residue was distributed into 50 ml of water and 50 ml of ethyl acetate, and a DCC urea material as an insoluble one was filtered off. An organic layer was extracted with 15 ml of a 5 % potassium carbonate aqueous solution twice, an aqueous layer was added. The aqueous layers were collected and acidified with concentrated hydrochloric acid and thereafter extracted with ethyl acetate. The extract was washed with a saturated sodium chloride aqueous solution and dried over sodium sulfate, and the solvent was distilled off to give 2.34 g of a crude product. The crude product was recrystallized from ethanol to give 1.39 g (yield 42 %) of 3,4,7-trimethyl-5-(1-ethyl-5-hydroxypyrazol-4-yl)carbonyl-2-hydrobenzo[b]thiophene-1,1-dioxide (Compound No. 82).

### Preparation Example 3

A 100-ml eggplant type flask was charged with 0.4 g (1.1 mmol) of the 3,3,4,7-tetramethyl-5-(1-ethyl-5-hydroxypyrazol-4-yl)carbonyl-2-hydrobenzo[b]thiophene-1,1-dioxide (Compound No. 94) obtained in Preparation Example 1, 10 ml of dichloromethane, 10 ml of water and 0.2 g (1.4 mmol) of potassium carbonate, and 0.19 g (1.3 mmol, 1.2 equivalents) of n-propanesulfonyl chloride was dropwise added at room temperature. 50 mg of benzyltriethylammonium chloride (BTEAC) was added, and the mixture was allowed to react at the above temperature for 2 hours. Then, the reaction mixture was liquid-separated. The resultant aqueous layer was extracted with dichloromethane, and the resultant organic layer was washed with a saturated sodium chloride aqueous solution and then dried over anhydrous sodium sulfate. The solvent was distilled off to give 0.45 g (yield 87 %) of 3,3,4,7-tetramethyl-5-(1-ethyl-5-n-propanesulfonyloxypyrazol-4-yl)carbonyl-2-hydrobenzo[b]thiophene-1,1-dioxide (Compound No. 95).

### Preparation Example 4

A 100-ml eggplant type flask was charged with 0.4 g (1.1 mmol) of 3,3,4,7-tetramethyl-5-(1-ethyl-5-hydroxypyrazol-4-yl)carbonyl-2-hydrobenzo[b]thiophene-1,1-dioxide, 10 ml of dichloromethane, 10 ml of water and 0.2 g (1.4 mmol, 1.3 equivalents) of potassium carbonate, and 0.25 g (1.3 mmol, 1.2 equivalents) of p-toluenesulfonic acid was dropwise added at room temperature. 50 mg of benzyltriethylammonium chloride (BTEAC) was added, and the mixture was allowed to react at the above temperature for 2 hours and then liquid-separated. The resultant aqueous layer was extracted with dichloromethane, and the resultant organic layer was washed with a saturated sodium chloride aqueous solution and dried over anhydrous sodium sulfate. The solvent was distilled off to give 0.52 g (yield 91 %) of 3,3,4,7-tetramethyl-5-(1-ethyl-5-p-toluenesulfonyloxypyrazol-4-yl)carbonyl-2-hydrobenzo[b]thiophene-5-carboxylic acid-1,1-dioxide (Compound No. 96).

### Preparation Example 5

A 30-ml eggplant type flask was charged with 0.30 g (0.86 mmol) of 3,4,7-trimethyl-5-(1-ethyl-5-hydroxypyrazol-4-yl)carbonyl-2-hydrobenzo[b]thiophene-1,1-dioxide, 5 ml of dichloromethane, 5 ml of water and 0.12 g (0.69 mmol, 0.8 equivalent) of potassium carbonate, and 0.16 g (0.95 mmol, 1.1 equivalents) of n-propanesulfonyl chloride was dropwise added with stirring at room temperature. 5 mg of benzyltriethylammonium chloride (BTEAC) was added, and the mixture was allowed to react at the above temperature for 2 hours and then liquid-separated. An aqueous layer was extracted with dichloromethane. An organic layer was washed with a saturated sodium chloride aqueous solution and dried over sodium sulfate. The solvent was distilled off to give 0.40 g of a crude product, and the crude product was recrystallized from ethanol to give 0.31 g (yield 79 %) of 3,4,7-trimethyl-5-(1-ethyl-5-n-propanesulfonyloxypyrazol-4-yl)-2-hydrobenzo[b]thiophenecarbonyl-1,1-dioxide (Compound No. 83).

### Preparation Example 6

A 30-ml eggplant type flask was charged with 0.25 g (0.72 mmol) of 3,4,7-trimethyl-5-(1-ethyl-5-hydroxypyrazol-4-yl)carbonyl-2-hydrobenzo[b]thiophene-1,1-dioxide, 5 ml of dichloromethane, 5 ml of water and 0.10 g (0.72 mmol, 1 equivalent) of potassium carbonate, and a solution of 0.15 g (0.72 mmol, 1.1 equivalents) of p-toluenesulfonyl chloride in 1 ml of dichloromethane was dropwise added with stirring at room temperature. 5 mg of benzyltriethylammonium chloride (BTEAC) was added, and the mixture was allowed to react at the above temperature for 2 hours and then liquid-separated. An aqueous layer was extracted with dichloromethane. An organic layer was washed with a saturated sodium chloride aqueous solution and dried over sodium sulfate. The solvent was distilled off to give 0.34 g of a crude product, and the crude product was recrystallized from ethanol to give 0.29 g (yield 80 %) of 3,4,7-trimethyl-5-(1-ethyl-p-toluenesulfonyloxypyrazol-4-yl)carbonyl-2-hydrobenzo[b]thiophene-1,1-dioxide (Compound No. 84).

### Preparation Example 7

A 30-ml eggplant type flask was charged with 0.30 g (0.86 mmol) of 3,4,7-trimethyl-5-(1-ethyl-5-hydroxypyrazol-4-yl)carbonyl-2-hydrobenzo[b]thiophene-1,1-dioxide, 5 ml of dichloromethane, 5 ml of water and 0.12 g (0.69 mmol, 0.8 equivalent) of potassium carbonate, and 0.12 g (0.95 mmol, 1.1 equivalents) of ethanesulfonyl chloride was dropwise added with stirring at room temperature. 5 mg of benzyltriethylammonium chloride (BTEAC) was added, and the mixture was allowed to react at the above temperature for 2 hours and then liquid-separated. An aqueous layer was extracted with dichloromethane. An organic layer was washed with a saturated sodium chloride aqueous solution and dried over sodium sulfate. The solvent was distilled off to give 0.35 g of a crude product, and the crude product was recrystallized from ethanol to give 0.24 g (yield 63 %) of 3,4,7-trimethyl-5-(1-ethyl-5-ethanesulfonyloxypyrazol-4-yl)-2-hydrobenzo[b]thiophenecarbonyl-1,1-dioxide (Compound No. 306).

### Preparation Example 8

A 30-ml eggplant type flask was charged with 0.30 g (0.86 mmol) of 3,4,7-trimethyl-5-(1-ethyl-5-hydroxypyrazol-4-yl)carbonyl-2-hydrobenzo[b]thiophene-1,1-dioxide, 5 ml of dichloromethane and 0.09 g (1.1 mmol, 1.3 equivalents) of pyridine. While the mixture was stirred with cooling with ice, 0.10 g (0.95 mmol, 1.1 equivalents) of isobutyric acid chloride was dropwise added. The mixture was stirred under cooling with ice for 30 minutes and then allowed to react at room temperature for 2 hours. The reaction was terminated by adding 5 ml of water, and then an aqueous layer was extracted with dichloromethane. An organic layer was washed with 5 % hydrochloric acid, with a 5 % potassium carbonate aqueous solution and then with a saturated sodium chloride aqueous solution and dried over sodium sulfate. The solvent was distilled off to give 0.28 g of a crude product, and the crude product was recrystallized from ethanol to give 0.27 g (yield 75 %) of 3,4,7-trimethyl-5-(1-ethyl-5-isopropylcarbonyloxypyrazol-4-yl)-2-hydrobenzo[b]-thiophenecarbonyl-1,1-dioxide (Compound No. 307).

### Preparation Example 9

A 30-ml eggplant type flask was charged with 0.24 g (0.69 mmol) of 3,4,7-trimethyl-5-(1-ethyl-5-hydroxypyrazol-4-yl)carbonyl-2-hydrobenzo[b]thiophene-1,1-dioxide, 5 ml of dichloromethane and 0.07 g (0.89 mmol, 1.3 equivalents) of pyridine. While the mixture was stirred with cooling with ice, 0.11 g (0.76 mmol, 1.1 equivalents) of cyclohexanecarboxylic acid chloride was dropwise added. The mixture was stirred under cooling with ice for 30 minutes and then allowed to react at room temperature for 2 hours. The reaction was terminated by adding 5 ml of water, and then an aqueous layer was extracted with dichloromethane. An organic layer was washed with 5 5 hydrochloric acid, with a 5 % potassium carbonate aqueous solution and then with a saturated sodium chloride aqueous solution and dried over sodium sulfate. The solvent was distilled off to give 0.35 of a crude product, and the crude product was recrystallized from ethanol to give 0.32 g (yield 100 %) of 3,4,7-trimethyl-5-(1-ethyl-5-carbonyloxypyrazol-4-yl)-2-hydrobenzo[b]thiophenecarbonyl-1,1-dioxide (Compound No. 308).

Table 2 shows the structural formulae of the starting materials or reaction reagents used in Preparation Examples 1 ∼ 9 and Compounds obtained in these Preparation Examples together with their yields, and Table 3 shows physical properties of Compounds obtained in Preparation Examples 1 ∼ 9.

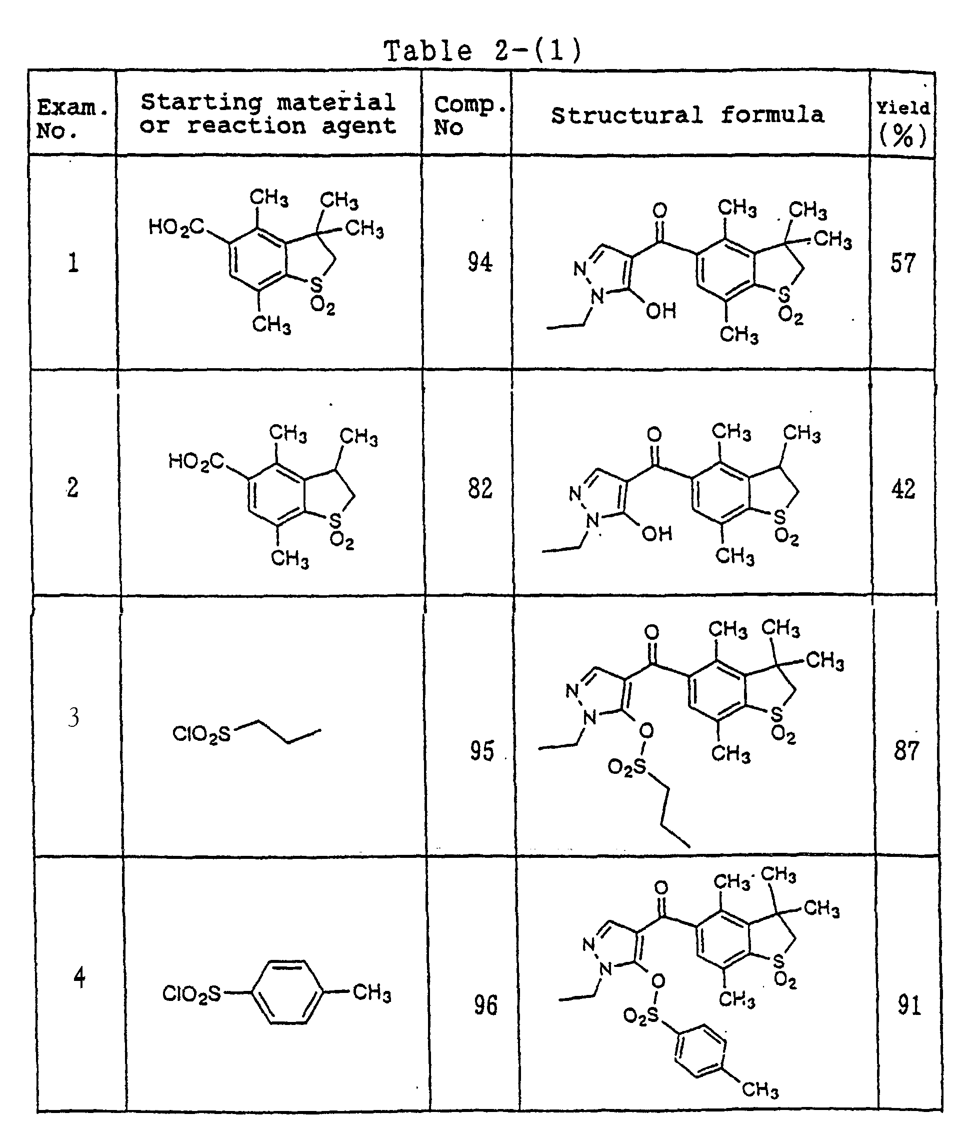

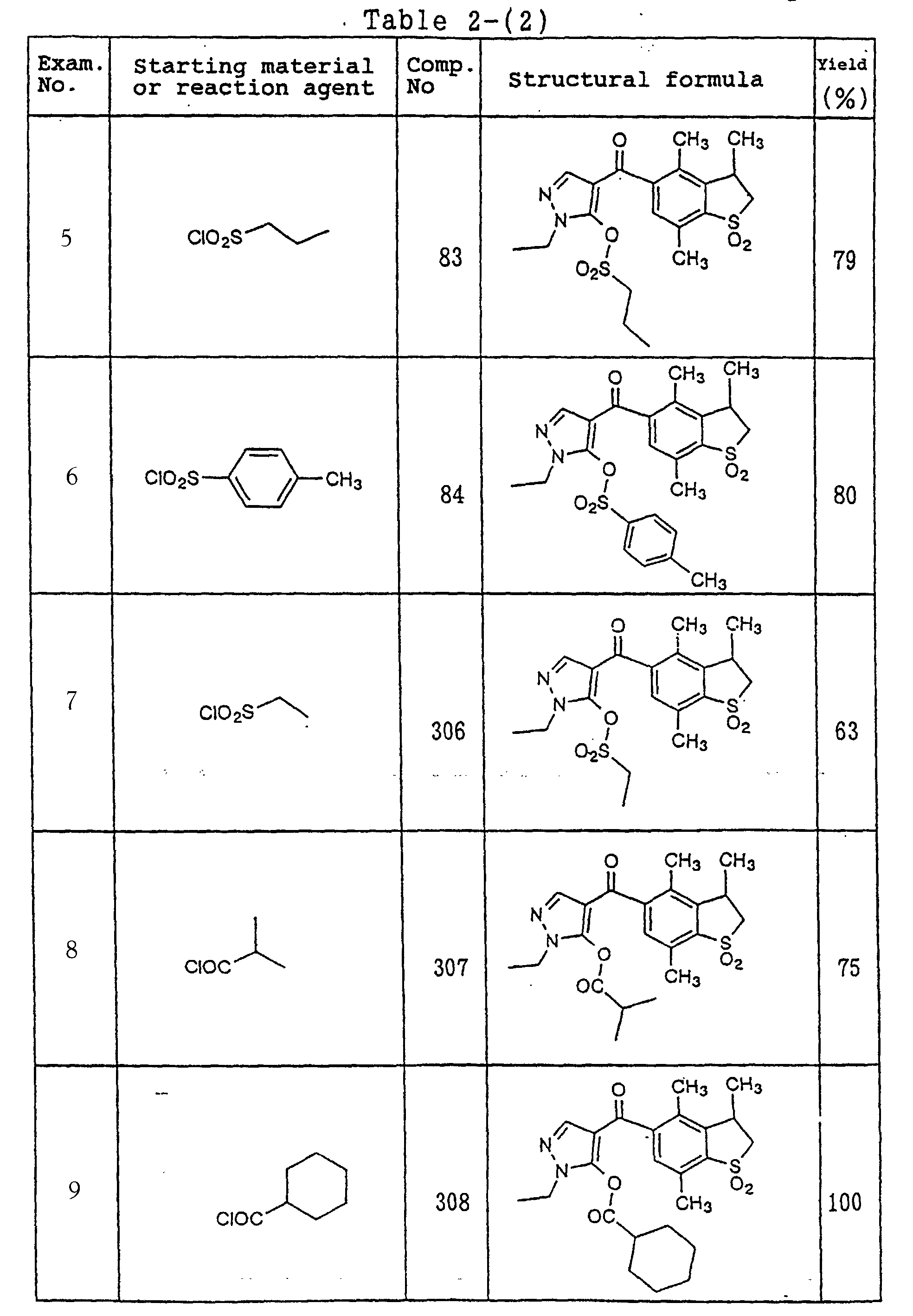

**Table 3-(1)**

| **Prep.** **Exam.** **No.** | **Comp.** **No.** | N M R (ppm) **Internal standard:** **tetramethylsilane** **Solvent: CDCl**_{**3**} | I R (cm⁻¹) |
|---|---|---|---|
| 1 | 94 | 1.46(3H,t) 1.67(6H,s) | 3000 |
| | | 2.45(3H,s) 2.64(3H,s) | 1660 |
| | | 3.37(2H,s) 4.08(2H,q) | 1305 |
| | | 4. 40(H, s) | 1100 |
| | | 7. 24(H, s) 7. 32(H, s) | |
| 2 | 82 | 1. 47(6H, t) 1. 49(3H, d) | 2980 |
| | | 2. 36(3H, s) 2. 65(3H, s) | 1640 |
| | | 3. 1∼3. 9(3H, m) 4. 09(2H, q) | 1295, 1175 |
| | | 6. 39(H, s) 7. 29(H, s) | 1120 |
| | | 7. 35(H, s) | |

**Table 3-(2)**

| **Prep** **Exam.** **No** | **Comp. No.** | NMR (ppm) **Internal standard:** **tetramethylsilane** **Solvent: CDCl**_{**3**} | I R(cm⁻¹) |
|---|---|---|---|
| 3 | 95 | 1. 19(3H, t) 1. 52(3H, t) | 2966 |
| | | 1. 65(3H, s) 1. 9∼2. 3(2H, m) | 1660 |
| | | 2. 39(3H, s) 2. 63(3H, s) | 1300 |
| | | 3. 37(2H, s) 3. 6∼3. 8(2H, m) | 1120 |
| | | 4. 29(2H, q) | |
| | | 7. 16(H, s) 7. 44(H, s) | |
| 4 | 96 | 1. 59(3H, t) 1. 65(6H, s) | 3000 |
| | | 2. 38(3H, s) 2. 48(3H, s) | 1670 |
| | | 2. 57(3H, s) 3. 36(2H, s) | 1305 |
| | | 4. 16(2h, q) 7. 20(H, s) | 1140 |
| | | 7.65(4H,dd) | |

**Table 3-(3)**

| **Prep.** **Exam. No**. | **Comp.** **No.** | N M R (ppm) **Internal standard:** **tetramethylsilane** **Solvent: CDCl**_{**3**} | I R(cm⁻¹) |
|---|---|---|---|
| 5 | 83 | 1.18(3H, t) | 3000 |
| | | 1. 44∼1. 6(6H, m) | 1670 |
| | | 1. 9∼2. 24(2H, m) 2. 29(3H, s) | 1400, 1310 |
| | | 2. 63(3H, s) 3. 2∼3. 8(5H, m) | 1185, 1135 |
| | | 4. 22(2H, q) | |
| | | 7. 20(H. s) 7. 45(H, s) | |
| 6 | 84 | 1. 41∼1. 63(6H, m) | 2990 |
| | | 2. 28(3H, s) 2. 47(3H, s) | 1660 |
| | | 2. 57(3H, s) 3. 2∼3. 8(3H, m) | 1370, 1300 |
| | | 4. 15(2H, q) 7. 06(H, s) | 1275, 1200 |
| | | 7. 37(H, s) 7. 67(4H, dd) | 1120 |

**Table 3-(4)**

| **Prep.** **Exam. No.** | **Comp.** **No.** | N M R (ppm) **Internal standard:** **tetramethylsilane** **Solvent: CDCl**_{**3**} | I R (cm⁻¹) |
|---|---|---|---|
| 7 | 306 | 1. 44∼1. 73(6H, m) | 3000 |
| | | 2. 29(3H, s) 2. 63(3H, s) | 1675 |
| | | 3. 2∼3. 89(5H, m) 4. 23(2H, q) | 1390, 1310 |
| | | 7. 20(H, s) 7. 45(H, s) | 1185, 1140 |
| 8 | 307 | 1. 28∼1. 52(12H, m) | 2990 |
| | | 2. 27(3H, s) 2. 61(3H, s) | 1790 |
| | | 2. 65∼2. 96(H, m) | 1660, 1300 |
| | | 3. 18∼3. 78(3H, m) | |
| | | 4. 02(2H, q) | |
| | | 7. 17(H, s) 7. 54(H, s) | |
| 9 | 308 | 1. 43(3H, t) 1. 47(3H, d) | 2950 |
| | | 2. 26(3H, s) 2. 61(3H, s) | 1780, 1660 |
| | | 1. 2∼2. 5(11H, m) | 1300 |
| | | 3. 2∼3. 8(3H, m) | |
| | | 4. 00(2H, q) | |
| | | 7. 16(H, s) 7. 58(1H, s) | |

Examples of the herbicide which achieves the second object of the present invention will be described hereinafter.

### Herbicide Examples and Herbicide Comparative Examples

### 1) Preparation of Herbicide

97 Parts by weight of talc (trade name: Zeaklite, supplied by Zeaklite Kogyo) as a carrier, 1.5 parts by weight of alkylarylsulfonate (trade name: Neoplex, supplied by Kao-Atlas K.K.) as a surfactant and 1.5 parts by weight of a nonionic and anionic surfactant (trade name: Sorpol 800A, supplied by Toho Chemical Co., Ltd.) were uniformly pulverized and mixed to prepare a carrier for a wettable powder.

90 Parts by weight of the above carrier and 10 parts by weight of one of the compounds of the present invention obtained in the above Preparation Examples were uniformly pulverized and mixed to obtain a herbicide. In Comparative Herbicide Examples 1 and 3, herbicides were similarly prepared using 10 parts by weight of the following compound (x), and in Comparative Herbicide Examples 2 and 4, herbicides were similarly prepared using 10 parts by weight of the following compound (y).

### Compound (x): Commercially available Pyrazolate

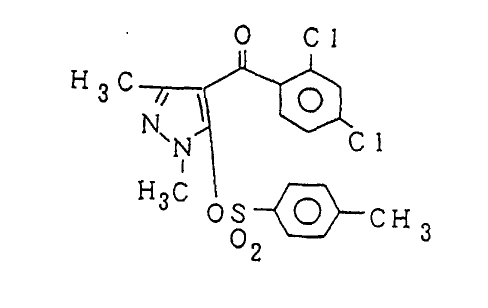

### Compound (y): Compound disclosed in JP-A-63-122672

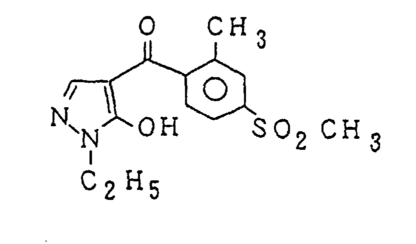

### (2) Biological test (Foliar treatment test, Herbicide Examples 1 ∼ 3 and Herbicide Comparative Examples 1 and 2)

Seeds of weeds such as large crabgrass, barnyardgrass, green foxtail, cocklebur, velvetleaf and slender amaranth and seeds of corn, wheat and barley were sown in 1/5,000-are Wagner pots filled with upland soil, and covered with upland soil. The seeds were grown in a greenhouse, and at the stage of 1 ∼ 2 leaves of these plants, a predetermined amount of the herbicide prepared in the above (1) was suspended in water and uniformly sprayed onto leaf and stalk portions at a rate of 200 liter/10 ares. Thereafter, the plants were grown in the greenhouse, and on 20th day after the treatment, the herbicide was evaluated for herbicidal efficacy. Table 4 shows the results.

The herbicidal efficacy and phytotoxcity to crops were shown on the basis of the following ratings.

The ratio of remaining plant weight to non-treated was determined on the basis of the ratio of remaining plant weight to non-treated = (remaining plant weight in treated plot/remaining plant weight in non-treated plot) x 100. This is also applicable to biological tests hereinafter.

| (Ratings) | |
|---|---|
| Herbicidal efficacy | Ratio of remaining plant weight to non-treated (%) |
| 0 | 81 - 100 |
| 1 | 61 - 80 |
| 2 | 41 - 60 |
| 3 | 21 - 40 |
| 4 | 1 - 20 |
| 5 | 0 |

| Phytotoxicity to crops | Ratio of remaining plant weight to non-treated (%) |
|---|---|
| - | 100 |
| ± | 95 - 99 |
| + | 90 - 94 |
| ++ | 80 - 89 |
| +++ | 0 - 79 |

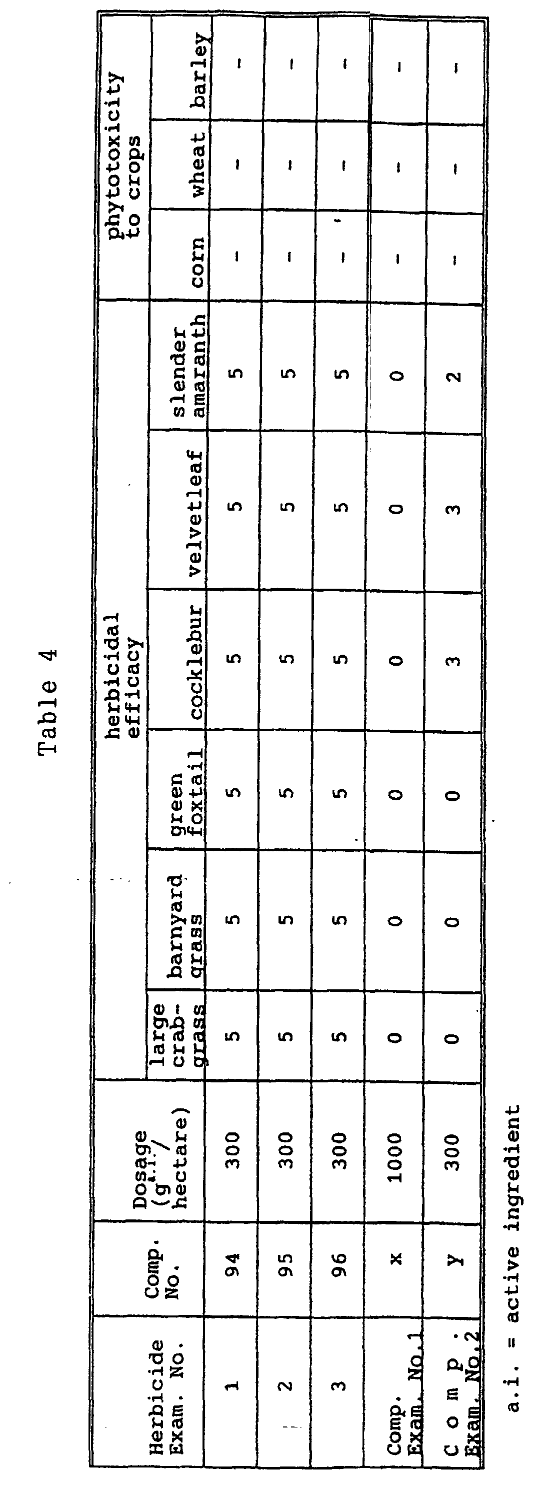

### (3) Biological test (Upland soil treatment test, Herbicide Examples 4-6 and Herbicide Comparative Examples 3 and 4)

Seeds of weeds such as large crabgrass, barnyardgrass, green foxtail, cocklebur, velvetleaf and slender amaranth and seeds of corn, wheat and barley were sown in 1/5,000-are Wagner pots filled with upland soil, and covered with upland soil. Then, a predetermined amount of the herbicide prepared in the above (1) was suspended in water and uniformly sprayed onto the soil surface. Thereafter, the plants were grown in the greenhouse, and on 20th day after the treatment, the herbicide was evaluated for herbicidal efficacy. Table 5 shows the results.

The herbicidal efficacy and the phytotoxicity to crops are shown on the basis of the ratings described in (2) Foliar treatment test.

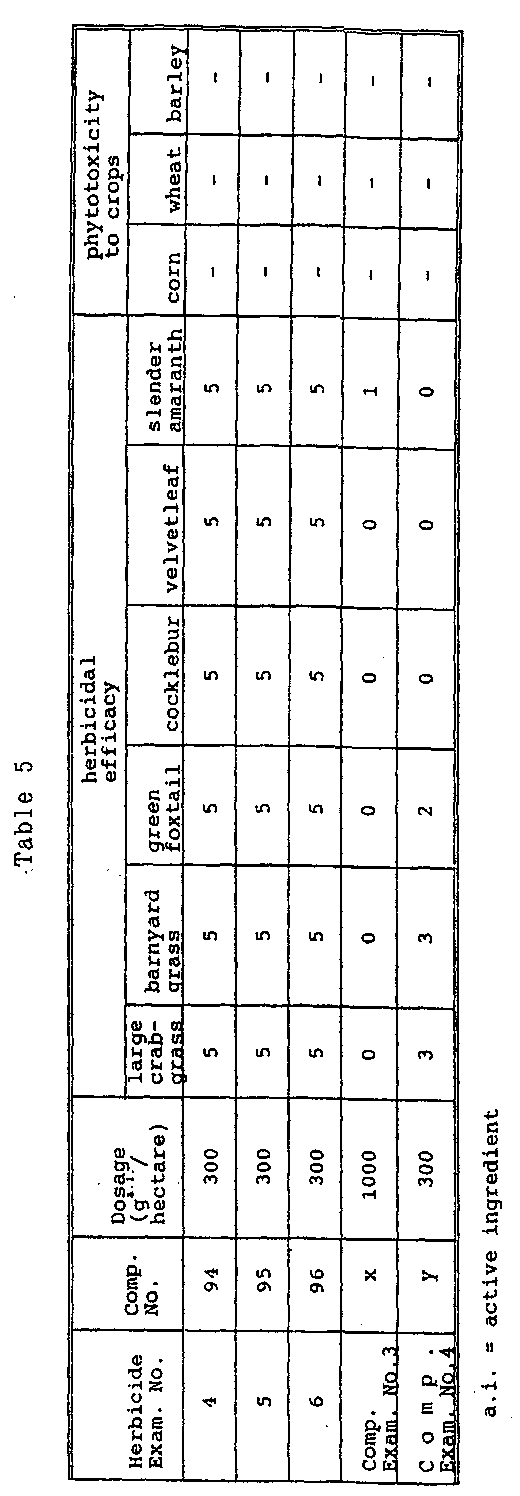

### (4) Biological test (Upland soil treatment test, Herbicide Examples 7-11 and Herbicide Comparative Example 5)

Seeds of weeds such as large crabgrass, barnyardgrass, green foxtail, cocklebur, velvetleaf and slender amaranth and seeds of corn were sown in 1/5,000-are Wagner pots filled with upland soil, and covered with upland soil. Then, a predetermined amount of the herbicide prepared in the above (1) was suspended in water and uniformly sprayed onto the soil surface. Thereafter, the plants were grown in the greenhouse, and on 20th day after the treatment, the herbicide was evaluated for herbicidal efficacy and phytotoxicity to corn. Table 6 shows the results.

The herbicidal efficacy and the phytotoxicity to crops are shown on the basis of the ratings described in (2) Foliar treatment test.

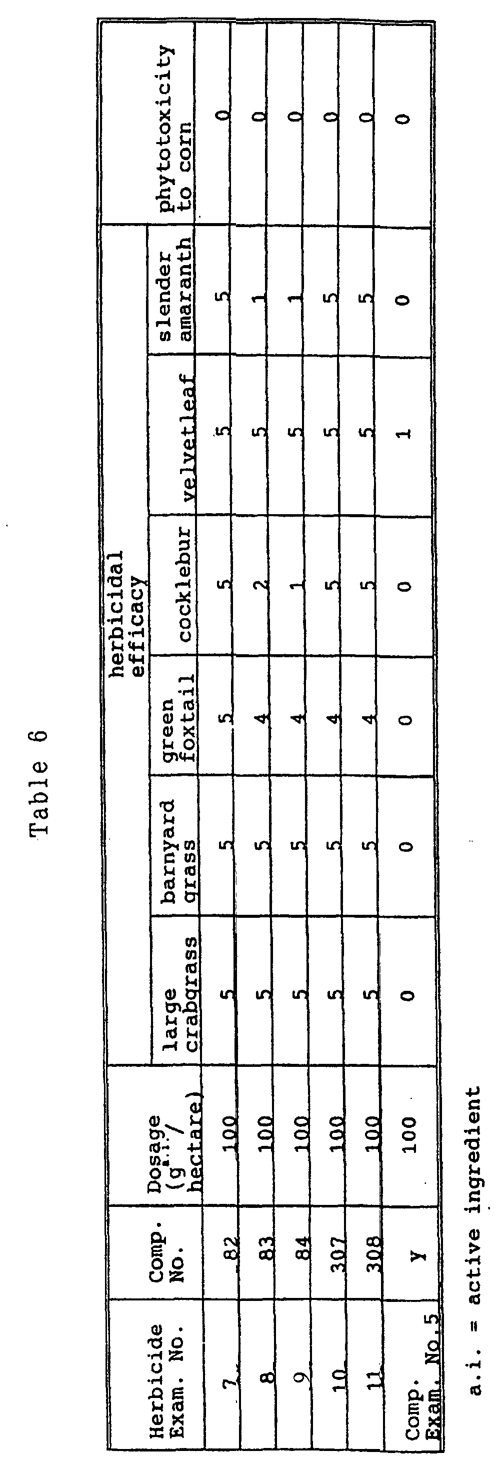

### Effect of the Invention

As explained in detail hereinabove, the present invention provides a novel pyrazole derivative which can selectively control gramineous weeds and broad-leaved weeds together at a low dosage by any one of foliar treatment and soil treatment without causing phytotoxicity on useful crops such as corn, wheat and barley, and a herbicide containing the novel pyrazole derivative as an active ingredient.

## Claims

1. A pyrazole derivative of the formula (I-A), 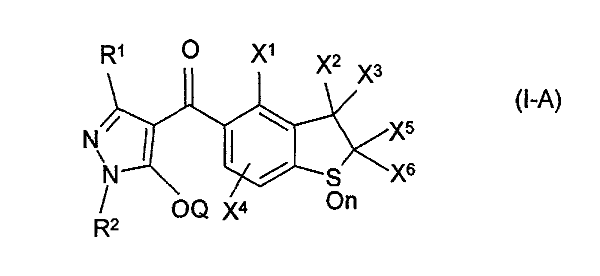 wherein:
R¹ is a hydrogen atom or a C₁-C₄ alkyl group;
R² is a C₁-C₄ alkyl group;
each of X² and X³ is independently one member selected from the group consisting of a hydrogen atom and a C₁-C₄ alkyl group;
each of X¹ and X⁴ is independently one member selected from the group consisting of a hydrogen atom, a halogen atom and a C₁-C₄ alkyl group;
each of X⁵ and X⁶ is independently a hydrogen atom or a C₁-C₄ alkyl group or X² and X⁵ may form an unsaturated bond by bonding to each other;
Q is a hydrogen atom or a group of -A-B in which A is one member selected from the group consisting of -SO₂-, -CO- and -CH₂CO-, and
B is one member selected from the group
consisting of a C₁-C₈ alkyl group, a C₃-C₈ cycloalkyl group and a group of the formula (V), 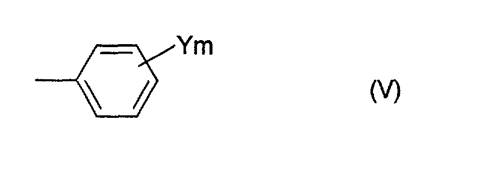 in which Y is one member selected from the group consisting of a halogen atom, a nitro group, a C₁-C₄ alkyl group, a C₁-C₄ alkoxy group and a C₁-C₄ haloalkyl group, and m is an integer of 0, 1 or 2 ; and n is 0, 1 or 2 , or a salt thereof.

2. The pyrazole derivative of the formula (I-A) or the salt thereof, as recited in Claim 1, wherein X² is a C₁-C₄ alkyl group, and X³ is a hydrogen atom or a C₁-C₄ alkyl group.

3. The pyrazole derivative of the formula (I-A) or the salt thereof, as recited in claim 1, wherein each of X² and X³ is independently a hydrogen atom or a methyl group.

4. The pyrazole derivative of the formula (I-A) or the salt thereof as recited in claim 3, wherein X² and/or X³ is methyl and X¹ is a C₁-C₄ alkyl group or a halogen atom.

5. The pyrazole derivative of the formula (I-A) or the salt thereof as recited in claim 1, wherein each of X¹ and X⁴ is independently a hydrogen atom, a halogen atom or a methyl group, X⁴ being in the 7 - position when it is a methyl group.

6. An aromatic carboxylic acid derivative of the formula (I I-A), 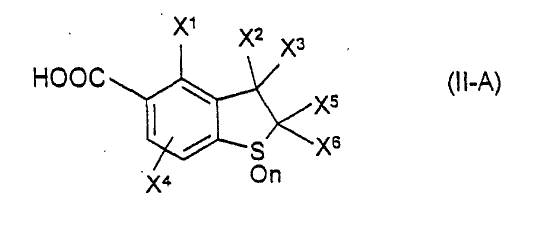 wherein:
each of X¹ and X⁴ is independently a halogen atom or a C₁-C₄ alkyl group;
each of X² and X³ is independently one member selected from the group consisting of a hydrogen atom and a C₁-C₄ alkyl group; each of X⁵ and X⁶ is independently a hydrogen atom or a C₁-C₄ alkyl group, or X² and X⁵ may form an unsaturated bond by bonding to each other; and n is 0, 1 or 2,
or a salt thereof.

7. A herbicide containing, as an active ingredient, the pyrazole derivative or its salt recited in any one of Claims 1 to 5.

## Patentansprüche

1. Pyrazolderivat der Formel (I-A) 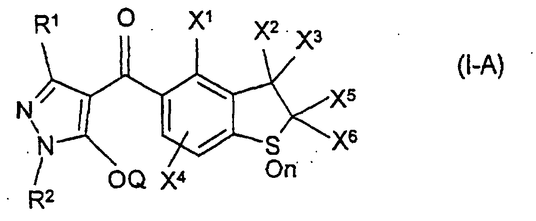 worin:
R¹ ein Wasserstoffatom oder eine C₁-C₄ Alkylgruppe ist;
R² eine C₁-C₄ Alkylgruppe ist;
X² und X³ jeweils unabhängig voneinander ein aus der Gruppe ausgewähltes Mitglied darstellen, die aus einem Wasserstoffatom und einer C₁-C₄ Alkylgruppe besteht;
X¹ und X⁴ jeweils unabhängig voneinander ein aus der Gruppe ausgewähltes Mitglied darstellen, die aus einem Wasserstoffatom, einem Halogenatom und einer C₁-C₄ Alkylgruppe besteht;
X⁵ und X⁶ jeweils unabhängig voneinander ein Wasserstoffatom oder eine C₁-C₄ Alkylgruppe sind oder X² und X⁵ können durch Bindung aneinander eine ungesättigte Bindung bilden;
Q ein Wasserstoffatom oder eine Gruppe von -A-B ist, worin A ein aus der Gruppe ausgewähltes Mitglied ist, die aus -SO₂-, -CO- und -CH₂CO- besteht, und B ein aus der Gruppe ausgewähltes Mitglied ist, die aus einer C₁-C₈ Alkylgruppe, einer C₃-C₈ Cycloalkylgruppe und einer Gruppe der Formel (V) besteht, 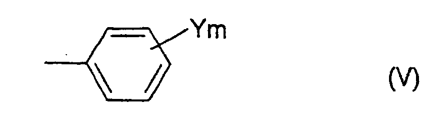
worin Y ein aus der Gruppe ausgewähltes Mitglied ist, die aus einem Halogenatom, einer Nitrogruppe, einer C₁-C₄ Alkylgruppe, C₁-C₄ Alkoxygruppe und einer C₁-C₄ Halogenalkylgruppe besteht, und m eine ganze Zahl von 0, 1 oder 2 ist und n 0, 1 oder 2 ist,
oder ein Salz davon.

2. Pyrazolderivat der Formel (I-A) oder ein Salz davon nach Anspruch 1, worin X² eine C₁-C₄ Alkylgruppe ist und X³ ein Wasserstoffatom oder eine C₁-C₄ Alkylgruppe ist.

3. Pyrazolderivat der Formel (I-A) oder ein Salz davon nach Anspruch 1, worin X² und X³ jeweils unabhängig voneinander ein Wasserstoffatom oder eine Methylgruppe sind.

4. Pyrazolderivat der Formel (I-A) oder ein Salz davon nach Anspruch 3, worin X² und/oder X³ eine Methylgruppe und X¹eine C₁-C₄ Alkylgruppe oder ein Halogenatom ist.

5. Pyrazolderivat der Formel (I-A) oder ein Salz davon nach Anspruch 1, worin X¹ und X⁴ jeweils unabhängig voneinander ein Wasserstoffatom, ein Halogenatom oder eine Methylgruppe sind, wobei X⁴ in der 7-Position vorliegt, wenn es eine Methylgruppe ist.

6. Aromatisches Carbonsäure-Derivat der Formel (II-A) 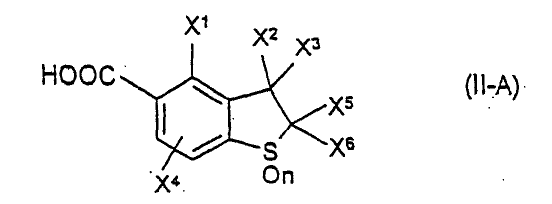 worin
X¹ und X⁴ jeweils unabhängig voneinander ein Halogenatom oder eine C₁-C₄ Alkylgruppe sind;
X² und X³ jeweils unabhängig voneinander ein aus der Gruppe ausgewähltes Mitglied darstellen, die aus einem Wasserstoffatom und einer C₁-C₄ Alkylgruppe besteht;
X⁵ und X⁶ jeweils unabhängig voneinander ein Wasserstoffatom oder eine C₁-C₄ Alkylgruppe sind oder X² und X⁵ können durch Bindung aneinander eine ungesättigte Bindung bilden; und
n ist 0, 1 oder 2,
oder ein Salz davon.

7. Herbizid, das als aktiven Wirkstoff das Pyrazolderivat oder ein Salz davon nach einem der Ansprüche 1 bis 5 enthält.

## Revendications

1. Dérivé de pyrazole de formule (I-A), 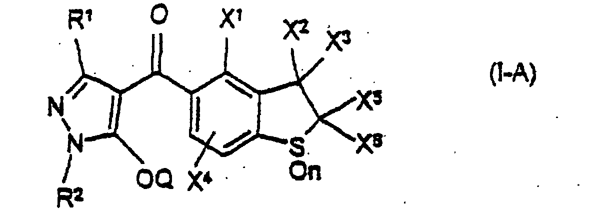 dans laquelle :
R¹ est un atome d'hydrogène ou un groupement alkyle en C₁ à C₄,
R² est un groupement alkyle en C₁ à C₄,
chacun de X² et de X³ est indépendamment un élément choisi parmi le groupe constitué d'un atome d'hydrogène et d'un groupement alkyle en C₁ à C₄,
chacun de X¹ et de X⁴ est indépendamment un élément choisi parmi le groupe constitué d'un atome d'hydrogène, d'un atome d'halogène et d'un groupement alkyle en C₁ à C₄,
chacun de X⁵ et de X⁶ est indépendamment un atome d'hydrogène ou un groupement alkyle en C₁ à C₄ ou bien
X² et X⁵ peuvent former une liaison insaturée en se liant l'un à l'autre,
Q est un atome d'hydrogène ou un groupement -A-B dans lequel A est un élément choisi parmi le groupe constitué de -SO₂-, -CO- et -CH₂CO-, et
B est un élément choisi parmi le groupe constitué d'un groupement alkyle en C₁ à C₈, d'un groupement cycloalkyle en C₃ à C₈ et d'un groupement de formule (V), 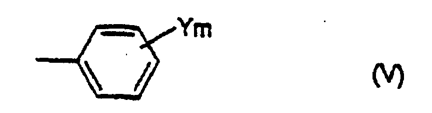
dans laquelle Y est un élément choisi parmi le groupe constitué d'un atome d'halogène, d'un groupement nitro, d'un groupement alkyle en C₁ à C₄, d'un groupement alcoxy en C₁ à C₄ et d'un groupement haloalkyle en C₁ à C₄, et m est un nombre entier de 0, 1 ou 2, et n vaut 0, 1 ou 2, ou sel de celui-ci.

2. Dérivé de pyrazole de formule (I-A) ou sel de celui-ci selon la revendication 1, dans lequel X² est un groupement alkyle en C₁ à C₄ et X³ est un atome d'hydrogène ou un groupement alkyle en C₁ à C₄.

3. Dérivé de pyrazole de formule (I-A) ou sel de celui-ci selon la revendication 1, dans lequel chacun de X² et de X³ est indépendamment un atome d'hydrogène ou un groupement méthyle.

4. Dérivé de pyrazole de formule (I-A) ou sel de celui-ci selon la revendication 3, dans lequel X² et/ou X³ est un méthyle et X¹ est un groupement alkyle en C₁ à C₄ ou un atome d'halogène.

5. Dérivé de pyrazole de formule (I-A) ou sel de celui-ci selon là revendication 1, dans lequel chacun de X¹ et de X⁴ est indépendamment un atome d'hydrogène, un atome d'halogène ou un groupement méthyle, X⁴ étant à la position 7 lorsqu'il s'agit d'un groupement méthyle.

6. Dérivé d'acide carboxylique aromatique de formule (II-A), 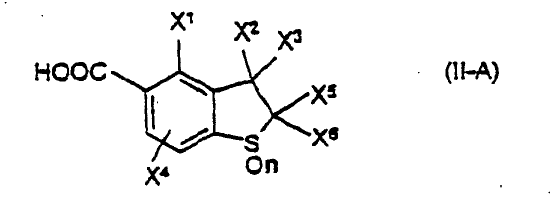 dans laquelle :
chacun de X¹ et de X⁴ est indépendamment un atome d'halogène ou un groupement alkyle en C₁ à C₄,
chacun de X² et de X³ est indépendamment un élément choisi parmi le groupe constitué d'un atome d'hydrogène et d'un groupement alkyle en C₁ à C₄,
chacun de X⁵ et de X⁶ est indépendamment un atome d'hydrogène ou un groupement alkyle en C₁ à C₄, ou bien
X² et X⁵ peuvent former une liaison insaturée en se liant l'un à l'autre, et n vaut 0, 1 ou 2,
ou sel de celui-ci.

7. Herbicide contenant, en tant que principe actif, le dérivé de pyrazole ou son sel selon l'une quelconque des revendications 1 à 5.
